(19) Europäisches Patentamt
European Patent Office
Office européen des brevets

(11) Veröffentlichungsnummer: **0 125 205**
**A1**

# (12) EUROPÄISCHE PATENTANMELDUNG

(21) Anmeldenummer: **84810180.4**

(22) Anmeldetag: **10.04.84**

(51) Int. Cl.[3]: **C 07 D 239/46**
C 07 D 251/16, C 07 D 251/46
C 07 C 143/78, C 07 C 143/828
C 07 C 143/83, A 01 N 47/36

(30) Priorität: **13.04.83 CH 1982/83**

(43) Veröffentlichungstag der Anmeldung:
**14.11.84 Patentblatt 84/46**

(84) Benannte Vertragsstaaten:
**AT BE CH DE FR GB IT LI NL**

(71) Anmelder: **CIBA-GEIGY AG**
**Postfach**
**CH-4002 Basel(CH)**

(72) Erfinder: **Meyer, Willy**
**Talweg 49**
**CH-4125 Riehen(CH)**

(72) Erfinder: **Oertle, Konrad, Dr.**
**Vogesenstrasse 10**
**CH-4106 Therwil(CH)**

(72) Erfinder: **Töpfl, Werner, Dr.**
**Dorneckstrasse 68**
**CH-4143 Dornach(CH)**

## (54) N-Phenylsulfonyl-N'-pyrimidinyl- und -triazinylharnstoffe.

(57) N-Phenylsulfonyl-N'-pyrimidinyl- und -triazinyl- harnstoffe der Formel I

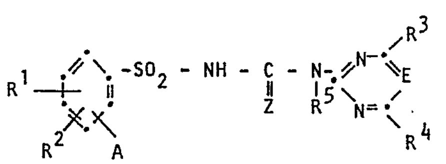

(I),

worin

A einen Rest der Formeln $-CR^6R^7-XR^8$, $-CR^9R^{10}R^{11}$ oder $-CHR^7-S-CO-R^{21}$,

$R^1$ Wasserstoff, Halogen, Nitro, Cyan, $C_1$-$C_4$-Alkyl, $C_1$/$C_4$-Halogen-alkyl, $-Y-R^{14}$, $-CO-NR^{12}R^{13}$, $-NR^{12}R^{13}$, $-SO-NR^{15}R^{16}$, $-O-SO_2-R^{17}$ oder $-CO-R^{18}$,

$R^2$ Wasserstoff, Halogen, $C_1$-$C_4$-Alkyl, $C_1$-$C_4$-Alkoxy, $C_1$-$C_4$-Alkythio, $C_1$-$C_4$-Alkysulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_5$-Alkoxyalkyl oder $-NR^{19}R^{20}$,

$R^5$ Wasserstoff, $C_1$-$C_4$-Alkyl oder $C_1$-$C_4$-Alkoxy,

$R^6$ Wasserstoff, $C_1$-$C_4$-Alkyl oder Fluor,

$R^7$ Wasserstoff oder methyl,

$R^8$ $C_1$-$C_4$-Halogenalkyl mit höchstens 2 Halogenatomen, $C_2$-$C_5$-Alkoxy-alkyl, $C_2$-$C_6$-Halogenalkenyl, $C_3$-$C_6$-Alkinyl oder $C_1$-$C_4$-Cyanoalkyl,

$R^9$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^{10}$ Wasserstoff, Halogen oder Methyl,

$R^{11}$ einen Rest $-CO-R^{24}$ oder einen einfach oder mehrfach durch Substituenten aus der Gruppe Cyan, Nitro, Hydroxyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$- Halogenalkylthio, $-CO-R^{18}$, $-S-CO-R^{18}$, $-O-CO-R^{18}$, $-NR^{12}R^{13}$, $-CO-NR^{12}R^{13}$, $-O-SO_2-R^{17}$ oder $-SO_2-NR^{15}R^{16}$ substituierten $C_1$-$C_4$-Alkylrest, der zusätzlich durch ein oder mehrere Halogenatome substituiert sein kann,

$R^{12}$ und $R^{13}$ unabhängig voneinander Wasserstoff oder $C_1$-$C_4$-Alkyl,

$R^{14}$ $C_2$-$C_5$-Alkenyl, $C_2$-$C_5$-Halogenalkenyl, $C_1$-$C_4$-Alkyl, $C_1$-$C_4$-Halogenalkyl oder $C_2$-$C_5$-Alkoxyalkyl,

$R^{15}$ Wasserstoff, $C_1$-$C_4$-Alkyl oder Methoxy,

$R^{16}$ Wasserstoff, Methyl oder Aethyl,

$R^{17}$ $C_1$-$C_4$-Alkyl, $C_1$-$C_4$-Halogenalkyl oder $-NR^{12}R^{13}$,

$R^{18}$ Wasserstoff, $C_1$-$C_4$-Alkoxy, $C_1$-$C_4$-Halogenalkoxy, $C_1$-$C_4$-Alkylthio, $C_1$-$C_4$-Alkyl, $C_1$-$C_4$-Halogenalkyl oder $C_2$-$C_5$-Alkoxyalkoxy,

./...

EP 0 125 205 A1

$R^{19}$ und $R^{20}$ unabhängig voneinander Wasserstoff oder $C_1$-$C_4$-Alkyl,

$R^{21}$ $C_1$-$C_4$-Alkyl, $C_1$-$C_4$-Alkoxy, $C_1$-$C_4$-Alkylthio, $-NR^{22}R^{23}$ oder gegebenenfalls durch Halogen oder $C_1$-$C_4$-Alkyl substituiertes Phenyl,

$R^{22}$ $C_1$-$C_4$-Alkyl oder Methoxy,

$R^{23}$ $C_1$-$C_4$-Alkyl,

$R^{22}$ und $R^{23}$ zusammen eine 4 bis 5 gliedrige, gegebenenfalls durch Sauerstoff oder $-NCH_3-$ unterbrochene Alkylenbrücke,

$R^{24}$ Wasserstoff, $C_1$-$C_4$-Alkyl oder $C_1$-$C_4$-Halogenalkyl,

E Stickstoff oder die Methylenbrücke,

Q und Z unabhängig voneinander Sauerstoff oder Schwefel und

Y und X unabhängig voneinander Sauerstoff, Schwefel, die Sulfinyl-oder Sulfonylbrücke bedeuten, sowie die Salze dieser Verbindungen.

CIBA-GEIGY AG
Basel (Schweiz)

5-14389/=

N-Phenylsulfonyl-N'-pyrimidinyl- und -triazinylharnstoffe

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-Phenylsulfonyl-N'-pyrimidinyl- und triazinylharnstoffe entsprechen der allgemeinen Formel

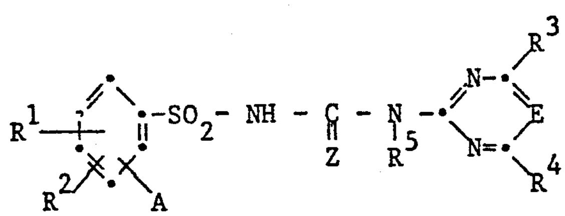
(I),

worin

A einen Rest der Formeln $-CR^6R^7-XR^8$, $-CR^9R^{10}R^{11}$ oder $-CHR^7-S-CQ-R^{21}$,

$R^1$ Wasserstoff, Halogen, Nitro, Cyan, $C_1$-$C_4$-Alkyl, $C_1$-$C_4$-Halogenalkyl, $-Y-R^{14}$, $-CO-NR^{12}R^{13}$, $-NR^{12}R^{13}$, $-SO-NR^{15}R^{16}$, $-O-SO_2-R^{17}$ oder $-CO-R^{18}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_5$-Alkoxyalkyl oder $-NR^{19}R^{20}$,

$R^5$ Wasserstoff, $C_1$-$C_4$-Alkyl oder $C_1$-$C_4$-Alkoxy,

$R^6$ Wasserstoff, $C_1$-$C_4$-Alkyl oder Fluor,

$R^7$ Wasserstoff oder Methyl,

$R^8$ $C_1$-$C_4$-Halogenalkyl mit höchstens 2 Halogenatomen, $C_2$-$C_5$-Alkoxyalkyl, $C_2$-$C_5$-Halogenalkenyl, $C_3$-$C_5$-Alkinyl oder $C_1$-$C_4$-Cyanoalkyl,

$R^9$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^{10}$ Wasserstoff, Halogen oder Methyl,

$R^{11}$ einen Rest $-CO-R^{24}$ oder einen einfach oder mehrfach durch Substituenten aus der Gruppe Cyan, Nitro, Hydroxyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $-CO-R^{18}$, $-S-CO-R^{18}$, $-O-CO-R^{18}$, $-NR^{12}R^{13}$, $-CO-NR^{12}R^{13}$, $-O-SO_2-R^{17}$ oder $-SO_2-NR^{15}R^{16}$ substituierten $C_1$-$C_4$-Alkylrest, der zusätzlich durch ein oder mehrere Halogenatome substituiert sein kann,

$R^{12}$ und $R^{13}$ unabhängig voneinander Wasserstoff oder $C_1$-$C_4$-Alkyl,

$R^{14}$ $C_2$-$C_5$-Alkenyl, $C_2$-$C_5$-Halogenalkenyl, $C_1$-$C_4$-Alkyl, $C_1$-$C_4$-Halogenalkyl oder $C_2$-$C_5$-Alkoxyalkyl,

$R^{15}$ Wasserstoff, $C_1$-$C_4$-Alkyl oder Methoxy,

$R^{16}$ Wasserstoff, Methyl oder Aethyl,

$R^{17}$ $C_1$-$C_4$-Alkyl, $C_1$-$C_4$-Halogenalkyl oder $-NR^{12}R^{13}$,

$R^{18}$ Wasserstoff, $C_1$-$C_4$-Alkoxy, $C_1$-$C_4$-Halogenalkoxy, $C_1$-$C_4$-Alkylthio, $C_1$-$C_4$-Alkyl, $C_1$-$C_4$-Halogenalkyl oder $C_2$-$C_5$-Alkoxyalkoxy,

$R^{19}$ und $R^{20}$ unabhängig voneinander Wasserstoff oder $C_1$-$C_4$-Alkyl,

$R^{21}$ $C_1$-$C_4$-Alkyl, $C_1$-$C_4$-Alkoxy, $C_1$-$C_4$-Alkylthio, $-NR^{22}R^{23}$ oder gegebenenfalls durch Halogen oder $C_1$-$C_4$-Alkyl substituiertes Phenyl,

$R^{22}$ $C_1$-$C_4$-Alkyl oder Methoxy,

$R^{23}$ $C_1$-$C_4$-Alkyl,

$R^{22}$ und $R^{23}$ zusammen eine 4 bis 5 gliedrige, gegebenenfalls durch Sauerstoff oder $-NCH_3-$ unterbrochene Alkylenbrücke,

$R^{24}$ Wasserstoff, $C_1$-$C_4$-Alkyl oder $C_1$-$C_4$-Halogenalkyl,

E Stickstoff oder die Methylenbrücke,

Q und Z unabhängig voneinander Sauerstoff oder Schwefel und

Y und X unabhängig voneinander Sauerstoff, Schwefel, die Sulfinyl-oder Sulfonylbrücke bedeuten, sowie die Salze dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentpublikationen Nr. 44210, 44807, 44808 und 44809 beschrieben.

In den Definitionen ist unter Alkyl geradkettiges oder verzweigtes Alkyl zu verstehen, z.B.: Methyl, Aethyl, n-Propyl, i-Propyl, die vier isomeren Butyl, n-Amyl, i-Amyl, 2-Amyl, 3-Amyl, n-Hexyl oder i-Hexyl. Bevorzugt sind Methyl und Aethyl.

Unter Alkoxy ist zu verstehen: Methoxy, Aethoxy, n-Propyloxy, i-Propyloxy und die vier isomeren Butyloxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Entsprechend sind unter Halogenalkyl bzw. Halogenalkylteilen von oben definierten Substituenten $R^1$ bis $R^{24}$ beispielsweise zu verstehen:

Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Unter die Definition des Substituenten A fallen beispielsweise die folgenden Reste: 2-Cyanoäthyl, 3-Cyanopropyl, 2-Cyanopropyl, 4-Cyanobutyl, 3-Cyanobutyl, 2-Acetoxyäthyl, 3-Acetoxypropyl, 2-Acetoxypropyl, 4-Acetoxybutyl, 3-Acetoxybutyl, 4-Acetoxypentyl, 2-Methoxycarbonyläthyl, 2-Methoxycarbonylpropyl, 3-Methoxycarbonylpropyl, 4-Methoxycarbonylbutyl, 3-Methoxycarbonylbutyl, 2-Aethoxycarbonyläthyl, 2-Aethoxycarbonylpropyl, 3-Aethoxycarbonylpropyl, 4-Aethoxycarbonylbutyl, 3-Aethoxycarbonylbutyl, 2-Methoxyäthyl, 3-Methoxypropyl, 2-Methoxypropyl, 4-Methoxybutyl, 3-Methoxybutyl, 4-Methoxypentyl, 2-Aethoxyäthyl, 3-Aethoxypropyl, 2-Aethoxypropyl, 4-Aethoxybutyl, 3-Aethoxybutyl, 4-Aethoxypentyl, 2-Methylthioäthyl, 3-Methylthiopropyl, 2-Methylthiopropyl, 4-Methylthiobutyl, 3-Methylthiobutyl, 4-Methylthiopentyl, 2-Hydroxyläthyl, 3-Hydroxylpropyl, 2-Hydroxylpropyl, 4-Hydroxylbutyl, 3-Hydroxylbutyl, 4-Hydroxylpentyl, 2-Methylcarbonyläthyl, 2-Aethylcarbonyläthyl, 2-Methylcarbonylpropyl, 3-Methylcarbonylpropyl, Difluormethoxymethyl, Difluormethoxyäthyl, (2-Methoxyäthoxy)-methyl, (2-Methoxyäthylthio)-methyl, Methylthiocarbonylthiomethyl, (2-Methylthioäthylthio)-methyl oder (2-Methylthioäthoxy)-methyl. Bevorzugt sind 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl, insbesondere aber 2-Aethoxycarbonyläthyl, 2-Aethoxycarbonylpropyl und 2-Cyanoäthyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin. Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel  sind die Verbindungen bevorzugt, in denen entweder

a) $R^1$ Wasserstoff ist oder
b) $R^2$ Wasserstoff ist oder
c) Z Sauerstoff ist oder
d) $R^5$ Wasserstoff ist oder
e) $R^3$ und $R^4$ zusammen höchstens 4 Kohlenstoffatome enthalten oder
f) der Rest A die 2-Stellung am Phenylring besetzt oder
g) $R^6$, $R^7$, $R^9$ und $R^{10}$ Wasserstoff bedeuten.

Weitere bevorzugte Gruppen von Wirkstoffen sind dadurch gekennzeichnet, dass in den Verbindungen der Formel I $R^1$, $R^2$ und $R^5$ für Wasserstoff und Z für Sauerstoff stehen und $R^3$ und $R^4$ zusammen höchstens 4 Kohlenstoffatomen enthalten.

Unter den Verbindungen der Untergruppe f) sind solche Verbindungen besonders bevorzugt, bei denen der Rest A ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

Eine weitere Bevorzugung in der oben genannten Gruppe besteht darin, dass die Reste $R^6$, $R^7$, $R^9$ und $R^{10}$ Wasserstoff bedeuten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

Eine ganz besonders bevorzugte Untergruppe von Wirkstoffen enthält solche Verbindungen der Formel I in denen die Reste $R^1$, $R^2$, $R^5$, $R^6$, $R^7$, $R^9$ und $R^{10}$ Wasserstoff und Z Sauerstoff bedeuten, die Reste $R^3$ und $R^4$ zusammen höchstens 4 Kohlenstoffatome enthalten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder

Methylthiocarbonylthiomethyl.

Als bevorzugte Einzelverbindung ist zu nennen:

N-[2-(3-Oxobutyl)-phenylsulfonyl]-N'-(4-methoxy-6-methyl-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

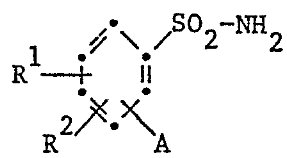

(II),

worin A, $R^1$ und $R^2$ die unter Formel I gegebene Bedeutung haben, in Gegenwart einer Base mit einem N-Pyrimidinyl- oder -Triazinylcarbamat der Formel III

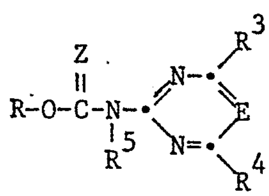

(III),

worin E, $R^3$, $R^4$, $R^5$ und Z die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein Phenylsulfonylisocyanat oder -isothiocyanat der Formel IV

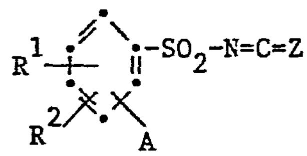

(IV),

worin A, $R^1$, $R^2$ und Z die unter Formel I gegebene Bedeutung haben, gegebenenfalls in Gegenwart einer Base, mit einem Amin der Formel V

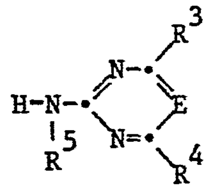

(V),

worin E, $R^3$, $R^4$ und $R^5$ die unter Formel I gegebene Bedeutung haben, umsetzt.

Nach einem weiteren Verfahren werden die Verbindungen der Formel I, in denen $R^5$ Wasserstoff bedeutet, hergestellt, indem man ein Sulfonamid der oben angegebenen Formel II gegebenenfalls in Gegenwart einer Base mit einem Isocyanat oder Isothiocyanat der Formel VI

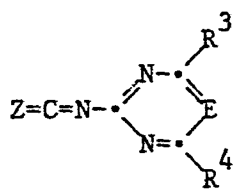

(VI),

worin E, $R^3$, $R^4$, $R^5$ und Z die unter Formel I gegebene Bedeutung haben, umsetzt.

Weiterhin kann man die Verbindungen der Formel I auch erhalten, indem man ein N-Phenylsulfonylcarbamat der Formel VII

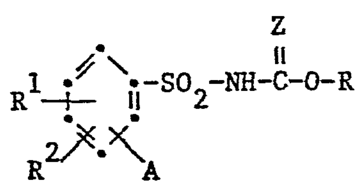

(VII),

worin A, $R^1$, $R^2$ und Z die unter Formel I gegebene Bedeutung haben, und R für Phenyl, Alkyl oder substituiertes Phenyl steht,

mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzung mit der äquimolaren Menge Base und Verdampfen des Lösungemittels.

Die Umsetzungen zu Verbindungen der Formel I werden vorteilhafterweise in aprotischen, inerten organischen Lösungsmittel vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durhcgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden. Als Basen sind insbesondere tertiäre Amine wie Trimethylamin, Triäthylamin, Chinuclidin, 1,4-Diazabicyclo-(2,2,2)-octan. 1,5-Diazabicyclo(4,3,0)non-5-en oder 1,5-Diazabicyclo(5,4,0)undec-7-en geeignet.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Ausgangsmaterialien III, V und VI sind aus der Literatur bekannt oder können nach bekannten Methoden hergestellt werden. Insbesondere können Verbindungen der Formeln III und VI nach bekannten Methoden aus den Verbindungen der Formel V erhalten werden.

Die Zwischenprodukte der Formeln II, IV und VII sind neu und wurden speziell für die Synthese der Verbindungen der Formel I entwickelt. Sie bilden daher einen Bestandteil der vorliegenden Erfindung.

Die neuen Zwischenprodukte der Formel II können auf verschiedenen Wegen hergestellt werden. So erhält man die Verbindungen der Formel II, indem man Aniline der Formel X

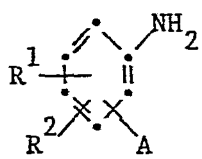

(X),

worin $R^1$, $R^2$ und A die unter Formel I gegebene Bedeutung haben, diazotiert und die Diazogruppe mit Schwefeldioxid in Gegenwart eines Katalysators wie Kupfer-I-chlorid in Salzsäure oder Essigsäure austauscht und das entstandene Phenylsulfonylchlorid mit Ammoniumhydroxid-Lösung umsetzt.

Ebenso kann man die Verbindungen der Formel II erhalten, indem man eine Phenylsulfonsäure der Formel XI

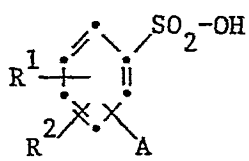

(XI),

worin $R^1$, $R^2$ und A die unter Formel I gegebene Bedeutung haben, durch Behandeln mit einem Chlorierungsmittel wie $PCl_5$, $POCl_3$, $COCl_2$ oder $SOCl_2$ in das entsprechende Phenylsulfonylchlorid überführt und dieses mit Ammoniumhydroxid-Lösung umsetzt.

Weiter kann man die Verbindungen der Formel II erhalten, indem man einen Benzylthioäther der Formel XII

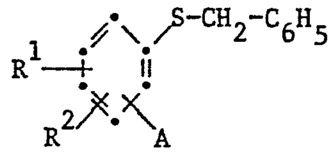

(XII),

worin $R^1$, $R^2$ und A die unter Formel I gegebene Bedeutung haben, durch Behandlung mit Chlor in das entsprechende Phenylsulfonylchlorid überführt und dieses mit Ammoniumhydroxid-Lösung umsetzt.

Die Verbindungen der Formel II, in denen A für substituiertes $C_2$-$C_6$-Alkyl steht können auch hergestellt werden, indem man ein Phenylsulfonamid der Formel XIII

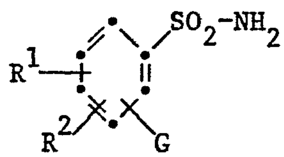

(XIII) ,

worin $R^1$ und $R^2$ die unter Formel I gegebene Bedeutung haben und G für einen substituierten ungesättigten $C_2$-$C_6$-Alkenyl- oder Alkinylrest steht, bis zur Sättigung hydriert.

Schliesslich kann man die Verbindungen der Formel II, in denen A für substituiertes $C_2$-$C_6$-Halogenalkyl steht, auch herstellen, indem man ein Phenylsulfonamid der Formel XIV

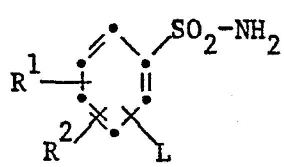

(XIV) ,

worin $R^1$ und $R^2$ die unter Formel I gegebene Bedeutung haben und L für einen substituierten ungesättigten $C_2$-$C_6$-Alkenyl- oder Alkinylrest steht, bis zur Sättigung halogeniert.

Das Halogenierungsverfahren wird mit Vorteil in der Siedehitze des Reaktionsgemisches durchgeführt. Als besonders vorteilhaft erweist sich hierbei die Bestrahlung der Reaktionslösung mit Licht oder der Zusatz eines Radikalkettenreaktionsstarters wie Dibenzoylperoxid oder α,α'-Azoisobutyronitril und die Verwendung eines chlorierten Kohlenwasserstoffs wie Methylenchlorid, Chloroform oder Tetrachlorkohlenstoff als Löungsmittel. Das Hydrierverfahren wird im allgemeinen in einem inerten Lösungsmittel wie Carbonsäuren, Alkohole, Ester oder Kohlenwasserstoffe unter einer Wasserstoffatmosphäre in Gegenwart von Hydrierkatalysatoren wie beispielsweise Raney-Nickel, Palladium- oder Platin-Katalysatoren durchgeführt.

Die ebenfalls neuen Phenylsulfonylisocyanate der Formel IV können beispielsweise durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in "Neuere Methoden der präparativen organischen Chemie", Band VI, 211-229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessende Umsetzung des Dikaliumsalzes mit Phosgen erhalten. Solche Verfahren sind in Arch. Pharm. 299, 174 (1966) beschrieben.

Die N-Phenylsulfonylcarbamate der Formel VII werden durch Umsetzung der Sulfonamide der Formel II mit Diphenylcarbonat in Gegenwart einer Base erhalten. Aehnliche Verfahren sind in der japanischen Patentschrift 61 169 erwähnt.

Die Ausgangsmaterialien der Formeln X, XI und XII sind allgemein bekannt oder sie können nach bekannten Verfahren hergestellt werden.

Die Verbindungen der Formel XIII sind in der europäischen Patentanmeldung 102 925 beschrieben, die der Formel XIV sind aus der europäischen Patentpublikation 44210 bekannt.

Die Verfahren zur Herstellung der Zwischenprodukte verlaufen im allgemeinen unter den gleichen Bedingungen wie sie für die Verfahren zur Herstellung der Endprodukte angegeben sind.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen herbiziden und Wuchsregulatoren - sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z.B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmittel eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische

oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen

Fettsäuregemischen, die z.B. aus Kokosnuss- oder Talgöl gewonnen werden können, genannt. Ferner sind auch die Fettsäure-methyl-taurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980-1981.

Die pestiziden Zubereitungen enthalten in der Regel 0,1 bis 95%, insbesondere 0,1 bis 80%, Wirkstoff der Formel I, 1 bis 99,9% eines

festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Emulgierbare Konzentrate

| | |
|---|---|
| Aktiver Wirkstoff: | 1 bis 20%, bevorzugt 5 bis 10% |
| oberflächenaktive Mittel: | 5 bis 30%, bevorzugt 10 bis 20% |
| flüssiges Trägermittel: | 50 bis 94%, bevorzugt 70 bis 85%. |

Stäube

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

Suspensions-Konzentrat

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bus 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelswerte eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,001 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:

Beispiel H1: N-[2-(2-Aethoxycarbonylpropyl)-phenylsulfonyl]-N'-(4-methoxy-6-methyl-pyrrimidin-2-yl)-harnstoff (Verbindung Nr. 2.115)

a) 2-(2-Aethoxycarbonylpropyl)-phenylsulfonsäure-Natriumsalz

Ein Gemisch von 84,6 g (0,46 Mol) Diazoniumsalz der Orthanilsäure, 200 ml Essigsäure, 37,7 g wasserfreiem Natriumacetat und 1,32 g Bis-(dibenzylidenaceton)-Palladium-Katalysator wird tropfenweise mit 55,1 g Methacrylsäureäthylester versetzt. Nach Abklingen der Reaktion wird das erhaltene Gemisch ohne Aufarbeitung nach Zusatz von 13 g eines 5 %igen Palladium/Kohle-Katalysators mit Wasserstoff hydriert. Anschliessend wird der Katalysator abgetrennt und durch Eindampfen der Lösung bis zur Trockne das 2-(2-Aethoxycarbonylpropyl/-phenylsulfonsäure-Natriumsalz isoliert. Ausbeute: 130 g.

b) 2-(2-Aethoxycarbonylpropyl)-phenylsulfonamid.

130 g 2-(2-Aethoxycarbonylpropyl)-phenylsulfonsäure-Natriumsalz werden in 250 ml Dimethylformamid gelöst und tropfenweise bei 20° - 30°C mit 79 ml Thionylchlorid versetzt. Das Gemisch wird für 2 Stunden bei 20° - 25°C gerührt, anschliessend auf Eiswasser gegossen und mit Aethylacetat extrahiert. Die organische Phase wird abgetrennt und mit einem Ueberschuss von 35 %-iger Ammoniaklösung versetzt. Dieses Gemisch wird für 0,5 Stunden gerührt.

Die abgetrennte organische Phase wird durch Chromatographie über Kieselgel mit Aethylacetat gereinigt. Man erhält so 44 g 2-(2-Aethoxycarbonylpropyl)-phenylsulfonamid, Smp. 70,5 - 71,5°C.

c) N-[2-(2-Aethoxycarbonylpropyl)-phenylsulfonyl]-N'-methyl-harnstoff.

Eine Lösung von 32 g 2-(2-Aethoxycarbonylpropyl)-phenylsulfonamid in 200 ml Acetonitril wird mit 6,8 g Methylisocyanat versetzt. Anschliessend werden 12 g Triäthylamin bei einer Temperatur von 20 - 25°C innerhalb von 10 Minuten tropfenweise zugesetzt. Nach 4 Stunden Rühren bei 20 - 25°C, Eingiessen der Lösung in Wasser und Ansäuern mit 10 %iger Salzsäure erhält man 30 g N-[2-(2-Aethoxycarbonylpropyl)-phenylsulfonyl]-N'-methyl-harnstoff vom Schmelzpunkt 141 - 143°C.

d) 2-(2-Aethoxycarbonylpropyl)-phenylsulfonylisocyanat.

22 g N-[2-(2-Aethoxycarbonylpropyl)-phenylsulfonyl]-N'-methyl-harnstoff werden in 100 ml Chlorbenzol gelöst. Dann werden bei einer Temperatur von 130°C innerhalb von 0,5 Stunden 20 g Phosgen eingeleitet und das Gemisch für eine weitere Stunde am Rückfluss gekocht. Durch Eindampfen und Fraktionieren des Rückstandes im Vakuum erhält man 16,2 g 2-(2-Aethoxycarbonylpropyl)-phenylsulfonylisocyanat, Sdp. 205°C/0,13 mb.

e) N-[2-(2-Aethoxycarbonylpropyl)-phenylsulfonyl]-N'-(4-methoxy-6-methyl-pyrimidin-2-yl)-harnstoff.

8,9 g 2-(2-Aethoxycarbonylpropyl)-phenylsulfonylisocyanat und 4,15 g 2-Amino-4-methoxy-6-methyl-pyrimidin werden in 25 ml absolutem Acetonitril 15 Stunden bei 20 - 25°C gerührt. Durch Abtrennen des ausgefallenen Niederschlages, Eindampfen der Lösung und Umkristallisieren des Rückstandes aus einem Aethylacetat/Hexan-Gemisch erhält man 8 g N-[2-(2-Aethoxycarbonylpropyl)-phenylsulfonyl]-N'-(4-methoxy-6-methyl-pyrimidin-2-yl)-harnstoff vom Schmelzpunkt 125°C.

Beispiel H2: N-[2-(3-Acetoxypropyl)-phenylsulfonyl]-N'-(4-methoxy-6-methyl-1,3,5-triazin-2-yl)-harnstoff (Verbindung Nr. 2.282)

a) Orthanilsäure-Diazoniumsalz

35,64 g (0,2 Mol) Orthanilsäure werden in 30 ml Wasser aufgeschlämmt, mit 62,3 ml Borfluorwasserstoffsäure (50 %ig; 0,5 Mol) versetzt und auf 0 - 5° C gekühlt. Innerhalb einer Stunde werden bei dieser Temperatur tropfenweise 13,8 g (0,2 Mol) Natriumnitrit, gelöst in 20 ml Wasser, unter Rühren zugetropft. Nach zusätzlichem Rühren während 30 Minuten werden 100 ml Diäthyläther zugegeben und die abgekühlte Suspension wird filtriert. Das isolierte feste Diazoniumsalz wird zusätzlich mit 100 ml eines 1:1 Gemisches aus Essigsäure und Diäthyläther und anschliessend mit nochmals 100 ml Diäthyläther gewaschen. Nach kurzem Trocknen an der Luft erhält man 34 g Diazoniumsalz; Ausbeute 92 % d.Th. Ohne Ausbeuteeinbusse kann anstelle von Borfluorwasserstoffsäure konzentrierte Salzsäure oder konzentrierte Schwefelsäure verwendet werden.

b) 2-(3-Acetoxy-1-propen-1-yl)-phenylsulfonsäure-Natriumsalz

Eine Mischung von 31,58 g Orthanilsäure-Diazoniumsalz, 14,1 g wasserfreiem Natriumacetat, 1,45 g Bis-(dibenzylidenaceton)-Palladium(0) und 100 ml Essigsäure wird tropfenweise mit 20,6 g Essigsäureallylester versetzt. Die Temperatur der Mischung steigt von 20° auf 30°C und wird durch externe Kühlung in einem Bereich von 30° - 35°C gehalten. Nach dem Abklingen der exothermen Reaktion wird das Gemisch für weitere 3 Stunden bei 30° - 35°C gerührt. Durch vollständiges Eindampfen der Reaktionsmischung erhält man 50 g rohes 2-(3-Acetoxy-1-propen-1-yl)-phenylsulfonsäure-Natriumsalz.

c) 2-(3-Acetoxypropyl)-phenylsulfonsäure-Natriumsalz

50 g rohes 2-(3-Acetoxy-1-propen-1-yl)-phenylsulfonsäure- Natriumsalz werden in 200 ml Essigsäure über 4,0 g 5 %iger Palladiumkohle unter einer Wasserstoffatmosphäre innerhalb innerhalb von 2 Stunden hydriert. Durch Abtrennen des Katalysators und vollständiges Eindampfen des Filtrats erhält man 50 g rohes 2-(3-Acetoxypropyl)-phenylsulfonsäure-Natriumsalz.

d) 2-(3-Acetoxypropyl)-phenylsulfonamid

50 g rohes 2-(3-Acetoxypropyl)-phenylsulfonsäure- Natriumsalz wird in 80 ml Dimethylformamid suspendiert und bei einer Temperatur von 20° - 25°C tropfenweise mit 31,2 g Thionylchlorid versetzt. Das Gemisch wird für 2 Stunden bei der gleichen Temperatur gerührt und anschliessend auf Eiswasser gegossen. Das ausgefallene 2-(3-Acetoxypropyl)-phenylsulfonylchlorid wird abgetrennt, in Aethylacetat gelöst und tropfenweise einer Mischung von 30 ml 30 %igem Ammoniak und Eis zugesetzt. Nach Beendigung der Reaktion wird das Gemisch mit Wasser verdünnt und mit Aethylacetat extrahiert. Die vereinigten organischen Phasen werden über Natriumsulfat getrocknet und vollständig eingedampft. Durch Säulenchromatographie an Kieselgel wird das erhaltene Rohprodukt gereinigt. Ausbeute: 9,0 g 2-(3-Acetoxypropyl)-phenylsulfonamid, Smp. 96° - 98°C.

e) N-[2-(3-Acetoxypropyl)-phenylsulfonyl]-N'-(4-methoxy-6-methyl-1,3,5-triazin-2-yl)-harnstoff

Eine Mischung aus 1,8 g 2-(3-Acetoxypropyl)-phenylsulfonamid, 1,82 g N-(4-Methoxy-6-methyl-1,3,5-triazin-2-yl)-phenylcarbamat, 1,07 g 1,5-Diazabicyclo[5,4,0]undec-5-en und 15 ml Dioxan wird für 3 Stunden bei einer Temperatur von 20° - 25°C gerührt. Zur Aufarbeitung wird das Reaktionsgemisch in Wasser eingegossen und mit 10 %iger Salzsäure angesäuert. Man erhält so 2,55 g N-[2-(3-Acetoxypropyl)-phenylsulfonyl]-N'-(4-methoxy-6-methyl-1,3,5-triazin-2-yl)-harnstoff, Smp. 134° - 135°C.

In analoger Weise erhält man die in den anschliessenden Tabellen aufgelisteten Zwischen- und Endprodukte.

Tabelle 1:

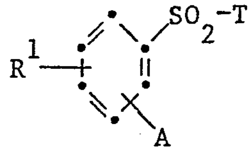

| Nr. | A | Stellung von A | $R^1$ | T | Phys. Daten |
|---|---|---|---|---|---|
| 1.1 | $-CH_2-CH_2-CH_2-CN$ | 2 | H | Cl | |
| 1.2 | $-CH_2-CH_2-CH_2-CN$ | 2 | H | $NH_2$ | |
| 1.3 | $-CH_2-CH_2-CH_2-CN$ | 2 | H | -N=C=O | |
| 1.4 | $-CH_2-CH_2-CH_2-CN$ | 2 | H | $-NH-CO-OC_6H_5$ | |
| 1.5 | $-CH_2-CH_2-CH_2-O-CO-CH_3$ | 2 | H | Cl | |
| 1.6 | $-CH_2-CH_2-CH_2-O-CO-CH_3$ | 2 | H | $NH_2$ | Smp.96-98°C |
| 1.7 | $-CH_2-CH_2-CH_2-O-CO-CH_3$ | 2 | H | -N=C=O | |
| 1.8 | $-CH_2-CH_2-CH_2-O-CO-CH_3$ | 2 | H | $-NH-CO-OC_6H_5$ | |
| 1.9 | $-CH_2-CH_2-COOCH_3$ | 2 | H | Cl | |
| 1.10 | $-CH_2-CH_2-COOCH_3$ | 2 | H | $NH_2$ | |
| 1.11 | $-CH_2-CH_2-COOCH_3$ | 2 | H | -N=C=O | |
| 1.12 | $-CH_2-CH_2-COOCH_3$ | 2 | H | $-NH-CO-OC_6H_5$ | |
| 1.13 | $-CH_2-CH(CH_3)-COOC_2H_5$ | 2 | H | Cl | |
| 1.14 | $-CH_2-CH(CH_3)-COOC_2H_5$ | 2 | H | $NH_2$ | Smp.70,5-71,5°C |
| 1.15 | $-CH_2-CH(CH_3)-COOC_2H_5$ | 2 | H | -N=C=O | Kp.205°C/ 0,13 mb |
| 1.16 | $-CH_2-CH(CH_3)-COOC_2H_5$ | 2 | H | $-NH-CO-OC_6H_5$ | |
| 1.17 | $-CH_2-CH_2-CH(CH_3)-O-CO-CH_3$ | 2 | H | Cl | |
| 1.18 | $-CH_2-CH_2-CH(CH_3)-O-CO-CH_3$ | 2 | H | $NH_2$ | |
| 1.19 | $-CH_2-CH_2-CH(CH_3)-O-CO-CH_3$ | 2 | H | -N=C=O | |
| 1.20 | $-CH_2-CH_2-CH(CH_3)-O-CO-CH_3$ | 2 | H | $-NH-CO-OC_6H_5$ | |
| 1.21 | $-CH_2-CH_2-CN$ | 2 | H | Cl | |
| 1.22 | $-CH_2-CH_2-CN$ | 2 | H | $NH_2$ | |
| 1.23 | $-CH_2-CH_2-CN$ | 2 | H | -N=C=O | |
| 1.24 | $-CH_2-CH_2-CN$ | 2 | H | $-NH-CO-OC_6H_5$ | |

Tabelle 1 (Fortsetzung)

| Nr. | A | Stellung von A | $R^1$ | T | Phys. Daten |
|---|---|---|---|---|---|
| 1.25 | $-CH_2-O-CH_2-CH_2-OCH_3$ | 2 | H | Cl | |
| 1.26 | $-CH_2-O-CH_2-CH_2-OCH_3$ | 2 | H | $NH_2$ | |
| 1.27 | $-CH_2-O-CH_2-CH_2-OCH_3$ | 2 | H | -N=C=O | |
| 1.28 | $-CH_2-O-CH_2-CH_2-OCH_3$ | 2 | H | $-NH-CO-OC_6H_5$ | |
| 1.29 | $-CH_2-S-CH_2-CH_2-OCH_3$ | 2 | H | Cl | |
| 1.30 | $-CH_2-S-CH_2-CH_2-OCH_3$ | 2 | H | $NH_2$ | |
| 1.31 | $-CH_2-S-CH_2-CH_2-OCH_3$ | 2 | H | -N=C=O | |
| 1.32 | $-CH_2-S-CH_2-CH_2-OCH_3$ | 2 | H | $-NH-CO-OC_6H_5$ | |
| 1.33 | $-CH_2-OCHF_2$ | 2 | H | Cl | |
| 1.34 | $-CH_2-OCHF_2$ | 2 | H | $NH_2$ | |
| 1.35 | $-CH_2-CH_2-OCH_3$ | 2 | H | $NH_2$ | Smp. 70°C (Zers.) |
| 1.36 | $-CH_2-CH_2-CH_2-OCH_3$ | 2 | H | $NH_2$ | |
| 1.37 | $-CH_2-CH_2-CH_2-OH$ | 2 | H | $NH_2$ | |
| 1.38 | $-CH_2-CH_2-CH(CH_3)-OH$ | 2 | H | $NH_2$ | |
| 1.39 | $-CH_2-CH_2-CO-CH_3$ | 2 | H | $NH_2$ | |
| 1.40 | $-CH_2-CH_2-COOC_2H_5$ | 2 | H | Cl | |
| 1.41 | $-CH_2-CH_2-COOC_2H_5$ | 2 | H | $NH_2$ | Smp.98-99°C |
| 1.42 | $-CH_2-CH_2-COOC_2H_5$ | 2 | H | -N=C=O | |
| 1.43 | $-CH_2-CH_2-COOC_2H_5$ | 2 | H | $-NH-CO-OC_6H_5$ | |
| 1.44 | $-CH(OC_2H_5)-CH_2-SO_2CH_3$ | 2 | H | $NH_2$ | |
| 1.45 | $-CH_2-S-CO-CH_3$ | 2 | H | $NH_2$ | Smp. 130-131°C |
| 1.46 | $-CH_2-S-CO-C_6H_5$ | 2 | H | $NH_2$ | Smp. 95-97°C |
| 1.47 | $-CH_2-S-CS-N(CH_3)_2$ | 2 | H | $NH_2$ | Smp. 124-125°C |
| 1.48 | $-CH_2-S-CS-N(CH_3)_2$ | 2 | H | $NH_2$ | Smp. 160-162°C |
| 1.49 | $-CH_2-S-CS-N(CH_3)OCH_3$ | 2 | H | $NH_2$ | Smp. 118-121°C |
| 1.50 | $-CH_2-S-CS-O-CH_3$ | 2 | H | $NH_2$ | Smp. 110-112°C |

Tabelle 2:

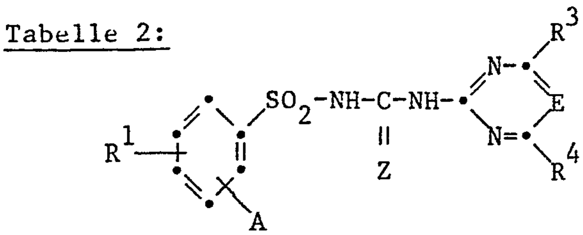

| Nr. | A | Stellung von A | $R^1$ | $R^3$ | $R^4$ | E | Z | Smp.[°C] |
|---|---|---|---|---|---|---|---|---|
| 2.1 | $-CH_2-CH_2-CN$ | 2 | H | $CH_3$ | $OCH_3$ | CH | O | 154-155 |
| 2.2 | $-CH_2-CH_2-CN$ | 2 | H | $CH_3$ | $OCHF_2$ | CH | O | |
| 2.3 | $-CH_2-CH_2-CN$ | 2 | H | $OCH_3$ | $OCH_3$ | CH | O | |
| 2.4 | $-CH_2-CH_2-CN$ | 2 | H | $OCH_3$ | $-N(CH_3)_2$ | CH | O | |
| 2.5 | $-CH_2-CH_2-CN$ | 2 | H | $CH_3$ | $CH_3$ | CH | O | |
| 2.6 | $-CH_2-CH_2-CN$ | 2 | H | $OCH_3$ | Cl | CH | O | |
| 2.7 | $-CH_2-CH_2-CN$ | 2 | H | $OCH_3$ | $OCHF_2$ | CH | O | |
| 2.8 | $-CH_2-CH_2-COOCH_3$ | 2 | H | $CH_3$ | $OCH_3$ | CH | O | |
| 2.9 | $-CH_2-CH_2-COOCH_3$ | 2 | H | $CH_3$ | $OCHF_2$ | CH | O | |
| 2.10 | $-CH_2-CH_2-COOCH_3$ | 2 | H | $CH_3$ | $CH_3$ | CH | O | |
| 2.11 | $-CH_2-CH_2-COOCH_3$ | 2 | H | $OCH_3$ | $OCH_3$ | CH | O | |
| 2.12 | $-CH_2-CH_2-COOCH_3$ | 2 | H | $OCH_3$ | Cl | CH | O | |
| 2.13 | $-CH_2-CH_2-COOCH_3$ | 2 | H | $OCH_3$ | $OCHF_2$ | CH | O | |
| 2.14 | $-CH_2-CH_2-COOCH_3$ | 2 | H | $OCH_3$ | $-N(CH_3)_2$ | CH | O | |
| 2.15 | $-CH_2-CH_2-CH_2-CN$ | 2 | H | $CH_3$ | $OCH_3$ | CH | O | |
| 2.16 | $-CH_2-CH_2-CH_2-CN$ | 2 | H | $CH_3$ | $OCHF_2$ | CH | O | |
| 2.17 | $-CH_2-CH_2-CH_2-CN$ | 2 | H | $OCH_3$ | $OCH_3$ | CH | O | |
| 2.18 | $-CH_2-CH_2-CH_2-CN$ | 2 | H | $OCH_3$ | $-N(CH_3)_2$ | CH | O | |
| 2.19 | $-CH_2-CH_2-CH_2-CN$ | 2 | H | $CH_3$ | $CH_3$ | CH | O | |
| 2.20 | $-CH_2-CH_2-CH_2-CN$ | 2 | H | $OCH_3$ | Cl | CH | O | |
| 2.21 | $-CH_2-CH_2-CH_2-CN$ | 2 | H | $OCH_3$ | $OCHF_2$ | CH | O | |
| 2.22 | $-CH_2-CH_2-CH_2-O-CO-CH_3$ | 2 | H | $CH_3$ | $OCH_3$ | CH | O | 159-161 |
| 2.23 | $-CH_2-CH_2-CH_2-O-CO-CH_3$ | 2 | H | $CH_3$ | $OCHF_2$ | CH | O | |

Tabelle 2: (Fortsetzung)

| Nr. | A | Stellung von A | $R^1$ | $R^3$ | $R^4$ | E | Z | Smp.[°C] |
|---|---|---|---|---|---|---|---|---|
| 2.24 | $-CH_2-CH_2-CH_2-O-CO-CH_3$ | 2 | H | $CH_3$ | $CH_3$ | CH | O | |
| 2.25 | $-CH_2-CH_2-CH_2-O-CO-CH_3$ | 2 | H | $OCH_3$ | $OCH_3$ | CH | O | |
| 2.26 | $-CH_2-CH_2-CH_2-O-CO-CH_3$ | 2 | H | $OCH_3$ | Cl | CH | O | |
| 2.27 | $-CH_2-CH_2-CH_2-O-CO-CH_3$ | 2 | H | $OCH_3$ | $OCHF_2$ | CH | O | |
| 2.28 | $-CH_2-CH_2-CH_2-O-CO-CH_3$ | 2 | H | $OCH_3$ | $-N(CH_3)_2$ | CH | O | |
| 2.29 | $-CH_2-OCH_2-CH_2-OCH_3$ | 2 | H | $CH_3$ | $OCH_3$ | CH | O | |
| 2.30 | $-CH_2-OCH_2-CH_2-OCH_3$ | 2 | H | $CH_3$ | $OC_2H_5$ | CH | O | |
| 2.31 | $-CH_2-OCH_2-CH_2-OCH_3$ | 2 | H | $CH_3$ | $OCHF_2$ | CH | O | |
| 2.32 | $-CH_2-OCH_2-CH_2-OCH_3$ | 2 | H | $OCH_3$ | Cl | CH | O | |
| 2.33 | $-CH_2-OCH_2-CH_2-OCH_3$ | 2 | H | $OCH_3$ | $OCHF_2$ | CH | O | |
| 2.34 | $-CH_2-OCH_2-CH_2-OCH_3$ | 2 | H | $OCH_3$ | $OCH_3$ | CH | O | |
| 2.35 | $-CH_2-OCH_2-CH_2-OCH_3$ | 2 | H | $OCH_3$ | $OC_2H_5$ | CH | O | |
| 2.36 | $-CH_2-OCH_2-CH_2-OCH_3$ | 2 | H | $OCH_3$ | $CH_2F$ | CH | O | |
| 2.37 | $-CH_2-OCH_2-CH_2-OCH_3$ | 2 | H | $OCH_3$ | $-N(CH_3)_2$ | CH | O | |
| 2.38 | $-CH_2-OCH_2-CH_2-OCH_3$ | 2 | H | $OCH_3$ | $-NHCH_3$ | CH | O | |
| 2.39 | $-CH_2-OCH_2-CH_2-OCH_3$ | 2 | H | $C_2H_5$ | $OCH_3$ | CH | O | |
| 2.40 | $-CH_2-OCH_2-CH_2-OCH_3$ | 2 | H | $C_2H_5$ | $OC_2H_5$ | CH | O | |
| 2.41 | $-CH_2-OCH_2-CH_2-OCH_3$ | 2 | H | $OCH_3$ | $-CH_2-OCH_3$ | CH | O | |
| 2.42 | $-CH_2-OCH_2-CH_2-OCH_3$ | 2 | H | $OCH_3$ | $-CH_2-OC_2H_5$ | CH | O | |
| 2.43 | $-CH_2-OCH_2-CH_2-OCH_3$ | 2 | H | $CH_3$ | $CH_3$ | CH | O | |
| 2.44 | $-CH_2-OCH_2-CH_2-OCH_3$ | 2 | H | $OCHF_2$ | $OCHF_2$ | CH | O | |
| 2.45 | $-CH_2-CH_2-CO-CH_3$ | 2 | H | $CH_3$ | $OCH_3$ | CH | O | |
| 2.46 | $-CH_2-CH_2-CO-CH_3$ | 2 | H | $CH_3$ | $OCHF_2$ | CH | O | |
| 2.47 | $-CH_2-CH_2-CO-CH_3$ | 2 | H | $OCH_3$ | $OCH_3$ | CH | O | |
| 2.48 | $-CH_2-CH_2-CO-CH_3$ | 2 | H | $OCH_3$ | $-N(CH_3)_2$ | CH | O | |
| 2.49 | $-CH_2-CH_2-CO-CH_3$ | 2 | H | $CH_3$ | $CH_3$ | CH | O | |
| 2.50 | $-CH_2-CH_2-CO-CH_3$ | 2 | H | $OCH_3$ | Cl | CH | O | |
| 2.51 | $-CH_2-CH_2-CO-CH_3$ | 2 | H | $OCH_3$ | $OCHF_2$ | CH | O | |

Tabelle 2: (Fortsetzung)

| Nr. | A | Stellung von A | $R^1$ | $R^3$ | $R^4$ | E | Z | Smp. [°C] |
|---|---|---|---|---|---|---|---|---|
| 2.52 | $-CH_2-CH_2-CH(OH)-CH_3$ | 2 | H | $CH_3$ | $OCH_3$ | CH | O | |
| 2.53 | $-CH_2-CH_2-CH(OH)-CH_3$ | 2 | H | $CH_3$ | $OCHF_2$ | CH | O | |
| 2.54 | $-CH_2-CH_2-CH(OH)-CH_3$ | 2 | H | $CH_3$ | $CH_3$ | CH | O | |
| 2.55 | $-CH_2-CH_2-CH(OH)-CH_3$ | 2 | H | $OCH_3$ | $OCH_3$ | CH | O | |
| 2.56 | $-CH_2-CH_2-CH(OH)-CH_3$ | 2 | H | $OCH_3$ | Cl | CH | O | |
| 2.57 | $-CH_2-CH_2-CH(OH)-CH_3$ | 2 | H | $OCH_3$ | $OCHF_2$ | CH | O | |
| 2.58 | $-CH_2-CH_2-CH(OH)-CH_3$ | 2 | H | $OCH_3$ | $-N(CH_3)_2$ | CH | O | |
| 2.59 | $-CH_2-CH_2-OH$ | 2 | H | $CH_3$ | $OCH_3$ | CH | O | |
| 2.60 | $-CH_2-CH_2-OH$ | 2 | H | $CH_3$ | $OCHF_2$ | CH | O | |
| 2.61 | $-CH_2-CH_2-OH$ | 2 | H | $OCH_3$ | $OCH_3$ | CH | O | |
| 2.62 | $-CH_2-CH_2-OH$ | 2 | H | $OCH_3$ | $-N(CH_3)_2$ | CH | O | |
| 2.63 | $-CH_2-CH_2-OH$ | 2 | H | $CH_3$ | $CH_3$ | CH | O | |
| 2.64 | $-CH_2-CH_2-OH$ | 2 | H | $OCH_3$ | Cl | CH | O | |
| 2.65 | $-CH_2-CH_2-OH$ | 2 | H | $OCH_3$ | $OCHF_2$ | CH | O | |
| 2.66 | $-CH_2-CH_2-CH_2-OH$ | 2 | H | $CH_3$ | $OCH_3$ | CH | O | |
| 2.67 | $-CH_2-CH_2-CH_2-OH$ | 2 | H | $CH_3$ | $OCHF_2$ | CH | O | |
| 2.68 | $-CH_2-CH_2-CH_2-OH$ | 2 | H | $CH_3$ | $CH_3$ | CH | O | |
| 2.69 | $-CH_2-CH_2-CH_2-OH$ | 2 | H | $OCH_3$ | $OCH_3$ | CH | O | |
| 2.70 | $-CH_2-CH_2-CH_2-OH$ | 2 | H | $OCH_3$ | Cl | CH | O | |
| 2.71 | $-CH_2-CH_2-CH_2-OH$ | 2 | H | $OCH_3$ | $OCHF_2$ | CH | O | |
| 2.72 | $-CH_2-CH_2-CH_2-OH$ | 2 | H | $OCH_3$ | $-N(CH_3)_2$ | CH | O | |
| 2.73 | $-CH_2-S-CS-N(CH_3)_2$ | 2 | H | $CH_3$ | $OCH_3$ | CH | O | 194-196° |
| 2.74 | $-CH_2-S-CS-N(CH_3)_2$ | 2 | H | $CH_3$ | $OCHF_2$ | CH | O | |
| 2.75 | $-CH_2-S-CS-N(CH_3)_2$ | 2 | H | $OCH_3$ | $OCH_3$ | CH | O | |
| 2.76 | $-CH_2-S-CS-N(CH_3)_2$ | 2 | H | $OCH_3$ | $-N(CH_3)_2$ | CH | O | |
| 2.77 | $-CH_2-S-CS-N(CH_3)_2$ | 2 | H | $CH_3$ | $CH_3$ | CH | O | |
| 2.78 | $-CH_2-S-CS-N(CH_3)_2$ | 2 | H | $OCH_3$ | Cl | CH | O | |
| 2.79 | $-CH_2-S-CS-N(CH_3)_2$ | 2 | H | $OCH_3$ | $OCHF_2$ | CH | O | |

Tabelle 2: (Fortsetzung)

| Nr. | A | Stellung von A | $R^1$ | $R^3$ | $R^4$ | E | Z | Smp.[°C] |
|---|---|---|---|---|---|---|---|---|
| 2.80 | $-CH_2-S-CO-CH_3$ | 2 | H | $CH_3$ | $OCH_3$ | CH | O | 191-194 (Zers.) |
| 2.81 | $-CH_2-S-CO-CH_3$ | 2 | H | $CH_3$ | $OCHF_2$ | CH | O | |
| 2.82 | $-CH_2-S-CO-CH_3$ | 2 | H | $CH_3$ | $CH_3$ | CH | O | |
| 2.83 | $-CH_2-S-CO-CH_3$ | 2 | H | $OCH_3$ | $OCH_3$ | CH | O | |
| 2.84 | $-CH_2-S-CO-CH_3$ | 2 | H | $OCH_3$ | Cl | CH | O | |
| 2.85 | $-CH_2-S-CO-CH_3$ | 2 | H | $OCH_3$ | $OCHF_2$ | CH | O | |
| 2.86 | $-CH_2-S-CO-CH_3$ | 2 | H | $OCH_3$ | $-N(CH_3)_2$ | CH | O | |
| 2.87 | $-CH_2-CH_2-OCH_3$ | 2 | H | $CH_3$ | $OCH_3$ | CH | O | 161-163 |
| 2.88 | $-CH_2-CH_2-OCH_3$ | 2 | H | $CH_3$ | $OCHF_2$ | CH | O | |
| 2.89 | $-CH_2-CH_2-OCH_3$ | 2 | H | $OCH_3$ | $OCH_3$ | CH | O | |
| 2.90 | $-CH_2-CH_2-OCH_3$ | 2 | H | $OCH_3$ | $-N(CH_3)_2$ | CH | O | |
| 2.91 | $-CH_2-CH_2-OCH_3$ | 2 | H | $CH_3$ | $CH_3$ | CH | O | |
| 2.92 | $-CH_2-CH_2-OCH_3$ | 2 | H | $OCH_3$ | Cl | CH | O | |
| 2.93 | $-CH_2-CH_2-OCH_3$ | 2 | H | $OCH_3$ | $OCHF_2$ | CH | O | |
| 2.94 | $-CH_2-CH_2-CH_2-OCH_3$ | 2 | H | $CH_3$ | $OCH_3$ | CH | O | |
| 2.95 | $-CH_2-CH_2-CH_2-OCH_3$ | 2 | H | $CH_3$ | $OCHF_2$ | CH | O | |
| 2.96 | $-CH_2-CH_2-CH_2-OCH_3$ | 2 | H | $CH_3$ | $CH_3$ | CH | O | |
| 2.97 | $-CH_2-CH_2-CH_2-OCH_3$ | 2 | H | $OCH_3$ | $OCH_3$ | CH | O | |
| 2.98 | $-CH_2-CH_2-CH_2-OCH_3$ | 2 | H | $OCH_3$ | Cl | CH | O | |
| 2.99 | $-CH_2-CH_2-CH_2-OCH_3$ | 2 | H | $OCH_3$ | $OCHF_2$ | CH | O | |
| 2.100 | $-CH_2-CH_2-CH_2-OCH_3$ | 2 | H | $OCH_3$ | $-N(CH_3)_2$ | CH | O | |
| 2.101 | $-CH_2-S-CH_2-CH_2-OCH_3$ | 2 | H | $CH_3$ | $OCH_3$ | CH | O | |
| 2.102 | $-CH_2-S-CH_2-CH_2-OCH_3$ | 2 | H | $CH_3$ | $OCHF_2$ | CH | O | |
| 2.103 | $-CH_2-S-CH_2-CH_2-OCH_3$ | 2 | H | $OCH_3$ | $OCH_3$ | CH | O | |
| 2.104 | $-CH_2-S-CH_2-CH_2-OCH_3$ | 2 | H | $OCH_3$ | $-N(CH_3)_2$ | CH | O | |
| 2.105 | $-CH_2-S-CH_2-CH_2-OCH_3$ | 2 | H | $CH_3$ | $CH_3$ | CH | O | |
| 2.106 | $-CH_2-S-CH_2-CH_2-OCH_3$ | 2 | H | $OCH_3$ | Cl | CH | O | |
| 2.107 | $-CH_2-S-CH_2-CH_2-OCH_3$ | 2 | H | $OCH_3$ | $OCHF_2$ | CH | O | |

Tabelle 2 (Fortsetzung)

| Nr. | A | Stellung von A | $R^1$ | $R^3$ | $R^4$ | E | Z | Smp.[°C] |
|---|---|---|---|---|---|---|---|---|
| 2.108 | $-CH_2-O-CHF_2$ | 2 | H | $CH_3$ | $OCH_3$ | CH | O | |
| 2.109 | $-CH_2-O-CHF_2$ | 2 | H | $CH_3$ | $OCHF_2$ | CH | O | |
| 2.110 | $-CH_2-O-CHF_2$ | 2 | H | $CH_3$ | $CH_3$ | CH | O | |
| 2.111 | $-CH_2-O-CHF_2$ | 2 | H | $OCH_3$ | $OCH_3$ | CH | O | |
| 2.112 | $-CH_2-O-CHF_2$ | 2 | H | $OCH_3$ | Cl | CH | O | |
| 2.113 | $-CH_2-O-CHF_2$ | 2 | H | $OCH_3$ | $OCHF_2$ | CH | O | |
| 2.114 | $-CH_2-O-CHF_2$ | 2 | H | $OCH_3$ | $-N(CH_3)_2$ | CH | O | |
| 2.115 | $-CH_2-CH(CH_3)-COOC_2H_5$ | 2 | H | $CH_3$ | $OCH_3$ | CH | O | 125 |
| 2.116 | $-CH_2-CH(CH_3)-COOC_2H_5$ | 2 | H | $CH_3$ | $OCHF_2$ | CH | O | |
| 2.117 | $-CH_2-CH(CH_3)-COOC_2H_5$ | 2 | H | $OCH_3$ | $OCH_3$ | CH | O | |
| 2.118 | $-CH_2-CH(CH_3)-COOC_2H_5$ | 2 | H | $OCH_3$ | $-N(CH_3)_2$ | CH | O | |
| 2.119 | $-CH_2-CH(CH_3)-COOC_2H_5$ | 2 | H | $CH_3$ | $CH_3$ | CH | O | |
| 2.120 | $-CH_2-CH(CH_3)-COOC_2H_5$ | 2 | H | $OCH_3$ | Cl | CH | O | |
| 2.121 | $-CH_2-CH(CH_3)-COOC_2H_5$ | 2 | H | $OCH_3$ | $OCHF_2$ | CH | O | |
| 2.122 | $-CH_2-CH_2-CH(CH_3)-O-CO-CH_3$ | 2 | H | $CH_3$ | $OCH_3$ | CH | O | |
| 2.123 | $-CH_2-CH_2-CH(CH_3)-O-CO-CH_3$ | 2 | H | $CH_3$ | $OCHF_2$ | CH | O | |
| 2.124 | $-CH_2-CH_2-CH(CH_3)-O-CO-CH_3$ | 2 | H | $CH_3$ | $CH_3$ | CH | O | |
| 2.125 | $-CH_2-CH_2-CH(CH_3)-O-CO-CH_3$ | 2 | H | $OCH_3$ | $OCH_3$ | CH | O | |
| 2.126 | $-CH_2-CH_2-CH(CH_3)-O-CO-CH_3$ | 2 | H | $OCH_3$ | Cl | CH | O | |
| 2.127 | $-CH_2-CH_2-CH(CH_3)-O-CO-CH_3$ | 2 | H | $OCH_3$ | $OCHF_2$ | CH | O | |
| 2.128 | $-CH_2-CH_2-CH(CH_3)-O-CO-CH_3$ | 2 | H | $OCH_3$ | $-N(CH_3)_2$ | CH | O | |
| 2.129 | $-CH_2-CH_2-CO-CH_3$ | 2 | H | $CH_3$ | $OCH_3$ | N | O | 153-154 |
| 2.130 | $-CH_2-CH_2-CO-CH_3$ | 2 | H | $CH_3$ | $OC_2H_5$ | N | O | |
| 2.131 | $-CH_2-CH_2-CO-CH_3$ | 2 | H | $OCH_3$ | $OCH_3$ | N | O | |
| 2.132 | $-CH_2-CH_2-CO-CH_3$ | 2 | H | $OCH_3$ | $OC_2H_5$ | N | O | |
| 2.133 | $-CH_2-CH_2-CO-CH_3$ | 2 | H | $OCH_3$ | $-N(CH_3)_2$ | N | O | |
| 2.134 | $-CH_2-CH_2-CO-CH_3$ | 2 | H | $OCH_3$ | $-NHCH_3$ | N | O | |
| 2.135 | $-CH_2-CH_2-CO-CH_3$ | 2 | H | $OCH_3$ | $-OCH_2-CF_3$ | N | O | |
| 2.136 | $-CH_2-CH_2-CO-CH_3$ | 2 | H | $OCH_3$ | $C_2H_5$ | N | O | |

Tabelle 2 (Fortsetzung)

| Nr. | A | Stellung von A | $R^1$ | $R^3$ | $R^4$ | E | Z | Smp.[°C] |
|---|---|---|---|---|---|---|---|---|
| 2.137 | $-CH_2-CH_2-CO-CH_3$ | 2 | H | $OC_2H_5$ | $C_2H_5$ | N | O | |
| 2.138 | $-CH_2-CH_2-CH(OH)-CH_3$ | 2 | H | $CH_3$ | $OCH_3$ | N | O | |
| 2.139 | $-CH_2-CH_2-CH(OH)-CH_3$ | 2 | H | $CH_3$ | $OC_2H_5$ | N | O | |
| 2.140 | $-CH_2-CH_2-CH(OH)-CH_3$ | 2 | H | $OCH_3$ | $OCH_3$ | N | O | |
| 2.141 | $-CH_2-CH_2-CH(OH)-CH_3$ | 2 | H | $OCH_3$ | $OC_2H_5$ | N | O | |
| 2.142 | $-CH_2-CH_2-CH(OH)-CH_3$ | 2 | H | $OCH_3$ | $-N(CH_3)_2$ | N | O | |
| 2.143 | $-CH_2-CH_2-CH(OH)-CH_3$ | 2 | H | $OCH_3$ | $-OCH_2-CF_3$ | N | O | |
| 2.144 | $-CH_2-CH_2-CH(OH)-CH_3$ | 2 | H | $OCH_3$ | $-NHCH_3$ | N | O | |
| 2.145 | $-CH_2-CH_2-CH(OH)-CH_3$ | 2 | H | $OCH_3$ | $C_2H_5$ | N | O | |
| 2.146 | $-CH_2-CH_2-CH(OH)-CH_3$ | 2 | H | $OC_2H_5$ | $C_2H_5$ | N | O | |
| 2.147 | $-CH_2-CH_2-OH$ | 2 | H | $CH_3$ | $OCH_3$ | N | O | |
| 2.148 | $-CH_2-CH_2-OH$ | 2 | H | $CH_3$ | $OC_2H_5$ | N | O | |
| 2.149 | $-CH_2-CH_2-OH$ | 2 | H | $OCH_3$ | $OCH_3$ | N | O | |
| 2.150 | $-CH_2-CH_2-OH$ | 2 | H | $OCH_3$ | $OC_2H_5$ | N | O | |
| 2.151 | $-CH_2-CH_2-OH$ | 2 | H | $OCH_3$ | $-N(CH_3)_2$ | N | O | |
| 2.152 | $-CH_2-CH_2-OH$ | 2 | H | $OCH_3$ | $-NHCH_3$ | N | O | |
| 2.153 | $-CH_2-CH_2-OH$ | 2 | H | $OCH_3$ | $-OCH_2-CF_3$ | N | O | |
| 2.154 | $-CH_2-CH_2-OH$ | 2 | H | $OCH_3$ | $C_2H_5$ | N | O | |
| 2.155 | $-CH_2-CH_2-OH$ | 2 | H | $OC_2H_5$ | $C_2H_5$ | N | O | |
| 2.156 | $-CH_2-CH_2-CH_2-OH$ | 2 | H | $CH_3$ | $OCH_3$ | N | O | |
| 2.157 | $-CH_2-CH_2-CH_2-OH$ | 2 | H | $CH_3$ | $OC_2H_5$ | N | O | |
| 2.158 | $-CH_2-CH_2-CH_2-OH$ | 2 | H | $OCH_3$ | $OCH_3$ | N | O | |
| 2.159 | $-CH_2-CH_2-CH_2-OH$ | 2 | H | $OCH_3$ | $OC_2H_5$ | N | O | |
| 2.160 | $-CH_2-CH_2-CH_2-OH$ | 2 | H | $OCH_3$ | $-N(CH_3)_2$ | N | O | |
| 2.161 | $-CH_2-CH_2-CH_2-OH$ | 2 | H | $OCH_3$ | $-OCH_2-CF_3$ | N | O | |
| 2.162 | $-CH_2-CH_2-CH_2-OH$ | 2 | H | $OCH_3$ | $-NHCH_3$ | N | O | |
| 2.163 | $-CH_2-CH_2-CH_2-OH$ | 2 | H | $OCH_3$ | $C_2H_5$ | N | O | |
| 2.164 | $-CH_2-CH_2-CH_2-OH$ | 2 | H | $OC_2H_5$ | $C_2H_5$ | N | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.165 | $-CH_2-S-CS-N(CH_3)_2$ | 2 | H | $CH_3$ | $OCH_3$ | N | O | 186-188 |
| 2.166 | $-CH_2-S-CS-N(CH_3)_2$ | 2 | H | $CH_3$ | $OC_2H_5$ | N | O | |
| 2.167 | $-CH_2-S-CS-N(CH_3)_2$ | 2 | H | $OCH_3$ | $OCH_3$ | N | O | |
| 2.168 | $-CH_2-S-CS-N(CH_3)_2$ | 2 | H | $OCH_3$ | $OC_2H_5$ | N | O | |
| 2.169 | $-CH_2-S-CS-N(CH_3)_2$ | 2 | H | $OCH_3$ | $-N(CH_3)_2$ | N | O | |
| 2.170 | $-CH_2-S-CS-N(CH_3)_2$ | 2 | H | $OCH_3$ | $-NHCH_3$ | N | O | |
| 2.171 | $-CH_2-S-CS-N(CH_3)_2$ | 2 | H | $OCH_3$ | $-OCH_2-CF_3$ | N | O | |
| 2.172 | $-CH_2-S-CS-N(CH_3)_2$ | 2 | H | $OCH_3$ | $C_2H_5$ | N | O | |
| 2.173 | $-CH_2-S-CS-N(CH_3)_2$ | 2 | H | $OC_2H_5$ | $C_2H_5$ | N | O | |
| 2.174 | $-CH_2-S-CS-CH_3$ | 2 | H | $CH_3$ | $OCH_3$ | N | O | |
| 2.175 | $-CH_2-S-CS-CH_3$ | 2 | H | $CH_3$ | $OC_2H_5$ | N | O | |
| 2.176 | $-CH_2-S-CS-CH_3$ | 2 | H | $OCH_3$ | $OCH_3$ | N | O | |
| 2.177 | $-CH_2-S-CS-CH_3$ | 2 | H | $OCH_3$ | $OC_2H_5$ | N | O | |
| 2.178 | $-CH_2-S-CS-CH_3$ | 2 | H | $OCH_3$ | $-N(CH_3)_2$ | N | O | |
| 2.179 | $-CH_2-S-CS-CH_3$ | 2 | H | $OCH_3$ | $-OCH_2-CF_3$ | N | O | |
| 2.180 | $-CH_2-S-CS-CH_3$ | 2 | H | $OCH_3$ | $-NHCH_3$ | N | O | |
| 2.181 | $-CH_2-S-CS-CH_3$ | 2 | H | $OCH_3$ | $C_2H_5$ | N | O | |
| 2.182 | $-CH_2-S-CS-CH_3$ | 2 | H | $OC_2H_5$ | $C_2H_5$ | N | O | |
| 2.183 | $-CH_2-CH_2-OCH_3$ | 2 | H | $CH_3$ | $OCH_3$ | N | O | 140-143 |
| 2.184 | $-CH_2-CH_2-OCH_3$ | 2 | H | $CH_3$ | $OC_2H_5$ | N | O | |
| 2.185 | $-CH_2-CH_2-OCH_3$ | 2 | H | $OCH_3$ | $OCH_3$ | N | O | |
| 2.186 | $-CH_2-CH_2-OCH_3$ | 2 | H | $OCH_3$ | $OC_2H_5$ | N | O | |
| 2.187 | $-CH_2-CH_2-OCH_3$ | 2 | H | $OCH_3$ | $-N(CH_3)_2$ | N | O | |
| 2.188 | $-CH_2-CH_2-OCH_3$ | 2 | H | $OCH_3$ | $-NHCH_3$ | N | O | |
| 2.189 | $-CH_2-CH_2-OCH_3$ | 2 | H | $OCH_3$ | $-OCH_2-CF_3$ | N | O | |
| 2.190 | $-CH_2-CH_2-OCH_3$ | 2 | H | $OCH_3$ | $C_2H_5$ | N | O | |
| 2.191 | $-CH_2-CH_2-OCH_3$ | 2 | H | $OC_2H_5$ | $C_2H_5$ | N | O | |
| 2.192 | $-CH_2-CH_2-CH_2-OCH_3$ | 2 | H | $CH_3$ | $OCH_3$ | N | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.193 | $-CH_2-CH_2-CH_2-OCH_3$ | 2 | H | $CH_3$ | $OC_2H_5$ | N | O | |
| 2.194 | $-CH_2-CH_2-CH_2-OCH_3$ | 2 | H | $OCH_3$ | $OCH_3$ | N | O | |
| 2.195 | $-CH_2-CH_2-CH_2-OCH_3$ | 2 | H | $OCH_3$ | $OC_2H_5$ | N | O | |
| 2.196 | $-CH_2-CH_2-CH_2-OCH_3$ | 2 | H | $OCH_3$ | $-N(CH_3)_2$ | N | O | |
| 2.197 | $-CH_2-CH_2-CH_2-OCH_3$ | 2 | H | $OCH_3$ | $-OCH_2-CF_3$ | N | O | |
| 2.198 | $-CH_2-CH_2-CH_2-OCH_3$ | 2 | H | $OCH_3$ | $-NHCH_3$ | N | O | |
| 2.199 | $-CH_2-CH_2-CH_2-OCH_3$ | 2 | H | $OCH_3$ | $C_2H_5$ | N | O | |
| 2.200 | $-CH_2-CH_2-CH_2-OCH_3$ | 2 | H | $OC_2H_5$ | $C_2H_5$ | N | O | |
| 2.201 | $-CH_2-S-CH_2-CH_2-OCH_3$ | 2 | H | $CH_3$ | $OCH_3$ | N | O | |
| 2.202 | $-CH_2-S-CH_2-CH_2-OCH_3$ | 2 | H | $CH_3$ | $OC_2H_5$ | N | O | |
| 2.203 | $-CH_2-S-CH_2-CH_2-OCH_3$ | 2 | H | $OCH_3$ | $OCH_3$ | N | O | |
| 2.204 | $-CH_2-S-CH_2-CH_2-OCH_3$ | 2 | H | $OCH_3$ | $OC_2H_5$ | N | O | |
| 2.205 | $-CH_2-S-CH_2-CH_2-OCH_3$ | 2 | H | $OCH_3$ | $-N(CH_3)_2$ | N | O | |
| 2.206 | $-CH_2-S-CH_2-CH_2-OCH_3$ | 2 | H | $OCH_3$ | $-NHCH_3$ | N | O | |
| 2.207 | $-CH_2-S-CH_2-CH_2-OCH_3$ | 2 | H | $OCH_3$ | $-OCH_2-CF_3$ | N | O | |
| 2.208 | $-CH_2-S-CH_2-CH_2-OCH_3$ | 2 | H | $OCH_3$ | $C_2H_5$ | N | O | |
| 2.209 | $-CH_2-S-CH_2-CH_2-OCH_3$ | 2 | H | $OC_2H_5$ | $C_2H_5$ | N | O | |
| 2.210 | $-CH_2-OCHF_2$ | 2 | H | $CH_3$ | $OCH_3$ | N | O | |
| 2.211 | $-CH_2-OCHF_2$ | 2 | H | $CH_3$ | $OC_2H_5$ | N | O | |
| 2.212 | $-CH_2-OCHF_2$ | 2 | H | $OCH_3$ | $OCH_3$ | N | O | |
| 2.213 | $-CH_2-OCHF_2$ | 2 | H | $OCH_3$ | $OC_2H_5$ | N | O | |
| 2.214 | $-CH_2-OCHF_2$ | 2 | H | $OCH_3$ | $-N(CH_3)_2$ | N | O | |
| 2.215 | $-CH_2-OCHF_2$ | 2 | H | $OCH_3$ | $-OCH_2-CF_3$ | N | O | |
| 2.216 | $-CH_2-OCHF_2$ | 2 | H | $OCH_3$ | $-NHCH_3$ | N | O | |
| 2.217 | $-CH_2-OCHF_2$ | 2 | H | $OCH_3$ | $C_2H_5$ | N | O | |
| 2.218 | $-CH_2-OCHF_2$ | 2 | H | $OC_2H_5$ | $C_2H_5$ | N | O | |
| 2.219 | $-CH_2-CH_2-COOC_2H_5$ | 2 | H | $CH_3$ | $OCH_3$ | N | O | |
| 2.220 | $-CH_2-CH_2-COOC_2H_5$ | 2 | H | $CH_3$ | $OC_2H_5$ | N | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.221 | $-CH_2-CH_2-COOC_2H_5$ | 2 | H | $OCH_3$ | $OCH_3$ | N | O | |
| 2.222 | $-CH_2-CH_2-COOC_2H_5$ | 2 | H | $OCH_3$ | $OC_2H_5$ | N | O | |
| 2.223 | $-CH_2-CH_2-COOC_2H_5$ | 2 | H | $OCH_3$ | $-N(CH_3)_2$ | N | O | |
| 2.224 | $-CH_2-CH_2-COOC_2H_5$ | 2 | H | $OCH_3$ | $-NHCH_3$ | N | O | |
| 2.225 | $-CH_2-CH_2-COOC_2H_5$ | 2 | H | $OCH_3$ | $-OCH_2-CF_3$ | N | O | |
| 2.226 | $-CH_2-CH_2-COOC_2H_5$ | 2 | H | $OCH_3$ | $C_2H_5$ | N | O | |
| 2.227 | $-CH_2-CH_2-COOC_2H_5$ | 2 | H | $OC_2H_5$ | $C_2H_5$ | N | O | |
| 2.228 | $-CH_2-CH_2-COOC_3H_7-i$ | 2 | H | $CH_3$ | $OCH_3$ | N | O | |
| 2.229 | $-CH_2-CH_2-COOC_3H_7-i$ | 2 | H | $CH_3$ | $OC_2H_5$ | N | O | |
| 2.230 | $-CH_2-CH_2-COOC_3H_7-i$ | 2 | H | $OCH_3$ | $OCH_3$ | N | O | |
| 2.231 | $-CH_2-CH_2-COOC_3H_7-i$ | 2 | H | $OCH_3$ | $OC_2H_5$ | N | O | |
| 2.232 | $-CH_2-CH_2-COOC_3H_7-i$ | 2 | H | $OCH_3$ | $-N(CH_3)_2$ | N | O | |
| 2.233 | $-CH_2-CH_2-COOC_3H_7-i$ | 2 | H | $OCH_3$ | $-OCH_2-CF_3$ | N | O | |
| 2.234 | $-CH_2-CH_2-COOC_3H_7-i$ | 2 | H | $OCH_3$ | $-NHCH_3$ | N | O | |
| 2.235 | $-CH_2-CH_2-COOC_3H_7-i$ | 2 | H | $OCH_3$ | $C_2H_5$ | N | O | |
| 2.236 | $-CH_2-CH_2-COOC_3H_7-i$ | 2 | H | $OC_2H_5$ | $C_2H_5$ | N | O | |
| 2.237 | $-CH_2-CH(CH_3)-COOC_2H_5$ | 2 | H | $CH_3$ | $OCH_3$ | N | O | 143 |
| 2.238 | $-CH_2-CH(CH_3)-COOC_2H_5$ | 2 | H | $CH_3$ | $OC_2H_5$ | N | O | |
| 2.239 | $-CH_2-CH(CH_3)-COOC_2H_5$ | 2 | H | $OCH_3$ | $OCH_3$ | N | O | |
| 2.240 | $-CH_2-CH(CH_3)-COOC_2H_5$ | 2 | H | $OCH_3$ | $OC_2H_5$ | N | O | |
| 2.241 | $-CH_2-CH(CH_3)-COOC_2H_5$ | 2 | H | $OCH_3$ | $-N(CH_3)_2$ | N | O | |
| 2.242 | $-CH_2-CH(CH_3)-COOC_2H_5$ | 2 | H | $OCH_3$ | $-NHCH_3$ | N | O | |
| 2.243 | $-CH_2-CH(CH_3)-COOC_2H_5$ | 2 | H | $OCH_3$ | $-OCH_2-CF_3$ | N | O | |
| 2.244 | $-CH_2-CH(CH_3)-COOC_2H_5$ | 2 | H | $OCH_3$ | $C_2H_5$ | N | O | |
| 2.245 | $-CH_2-CH(CH_3)-COOC_2H_5$ | 2 | H | $OC_2H_5$ | $C_2H_5$ | N | O | |
| 2.246 | $-CH_2-CH_2-CH(CH_3)-O-CO-CH_3$ | 2 | H | $CH_3$ | $OCH_3$ | N | O | |
| 2.247 | $-CH_2-CH_2-CH(CH_3)-O-CO-CH_3$ | 2 | H | $CH_3$ | $OC_2H_5$ | N | O | |
| 2.248 | $-CH_2-CH_2-CH(CH_3)-O-CO-CH_3$ | 2 | H | $OCH_3$ | $OCH_3$ | N | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.249 | $-CH_2-CH_2-CH(CH_3)-O-CO-CH_3$ | 2 | H | $OCH_3$ | $OC_2H_5$ | N | O | |
| 2.250 | $-CH_2-CH_2-CH(CH_3)-O-CO-CH_3$ | 2 | H | $OCH_3$ | $-N(CH_3)_2$ | N | O | |
| 2.251 | $-CH_2-CH_2-CH(CH_3)-O-CO-CH_3$ | 2 | H | $OCH_3$ | $-OCH_2-CF_3$ | N | O | |
| 2.252 | $-CH_2-CH_2-CH(CH_3)-O-CO-CH_3$ | 2 | H | $OCH_3$ | $-NHCH_3$ | N | O | |
| 2.253 | $-CH_2-CH_2-CH(CH_3)-O-CO-CH_3$ | 2 | H | $OCH_3$ | $C_2H_5$ | N | O | |
| 2.254 | $-CH_2-CH_2-CH(CH_3)-O-CO-CH_3$ | 2 | H | $OC_2H_5$ | $C_2H_5$ | N | O | |
| 2.255 | $-CH_2-CH_2-CN$ | 2 | H | $CH_3$ | $OCH_3$ | N | O | |
| 2.256 | $-CH_2-CH_2-CN$ | 2 | H | $CH_3$ | $OC_2H_5$ | N | O | |
| 2.257 | $-CH_2-CH_2-CN$ | 2 | H | $OCH_3$ | $OCH_3$ | N | O | |
| 2.258 | $-CH_2-CH_2-CN$ | 2 | H | $OCH_3$ | $OC_2H_5$ | N | O | |
| 2.259 | $-CH_2-CH_2-CN$ | 2 | H | $OCH_3$ | $-N(CH_3)_2$ | N | O | |
| 2.260 | $-CH_2-CH_2-CN$ | 2 | H | $OCH_3$ | $-NHCH_3$ | N | O | |
| 2.261 | $-CH_2-CH_2-CN$ | 2 | H | $OCH_3$ | $-OCH_2-CF_3$ | N | O | |
| 2.262 | $-CH_2-CH_2-CN$ | 2 | H | $OCH_3$ | $C_2H_5$ | N | O | |
| 2.263 | $-CH_2-CH_2-CN$ | 2 | H | $OC_2H_5$ | $C_2H_5$ | N | O | |
| 2.264 | $-CH_2-CH_2-COOCH_3$ | 2 | H | $CH_3$ | $OCH_3$ | N | O | |
| 2.265 | $-CH_2-CH_2-COOCH_3$ | 2 | H | $CH_3$ | $OC_2H_5$ | N | O | |
| 2.266 | $-CH_2-CH_2-COOCH_3$ | 2 | H | $OCH_3$ | $OCH_3$ | N | O | |
| 2.267 | $-CH_2-CH_2-COOCH_3$ | 2 | H | $OCH_3$ | $OC_2H_5$ | N | O | |
| 2.268 | $-CH_2-CH_2-COOCH_3$ | 2 | H | $OCH_3$ | $-N(CH_3)_2$ | N | O | |
| 2.269 | $-CH_2-CH_2-COOCH_3$ | 2 | H | $OCH_3$ | $-OCH_2-CF_3$ | N | O | |
| 2.270 | $-CH_2-CH_2-COOCH_3$ | 2 | H | $OCH_3$ | $-NHCH_3$ | N | O | |
| 2.271 | $-CH_2-CH_2-COOCH_3$ | 2 | H | $OCH_3$ | $C_2H_5$ | N | O | |
| 2.272 | $-CH_2-CH_2-COOCH_3$ | 2 | H | $OC_2H_5$ | $C_2H_5$ | N | O | |
| 2.273 | $-CH_2-CH_2-CH_2-CN$ | 2 | H | $CH_3$ | $OCH_3$ | N | O | |
| 2.274 | $-CH_2-CH_2-CH_2-CN$ | 2 | H | $CH_3$ | $OC_2H_5$ | N | O | |
| 2.275 | $-CH_2-CH_2-CH_2-CN$ | 2 | H | $OCH_3$ | $OCH_3$ | N | O | |
| 2.276 | $-CH_2-CH_2-CH_2-CN$ | 2 | H | $OCH_3$ | $OC_2H_5$ | N | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.277 | $-CH_2-CH_2-CH_2-CN$ | 2 | H | $OCH_3$ | $-N(CH_3)_2$ | N | O | |
| 2.278 | $-CH_2-CH_2-CH_2-CN$ | 2 | H | $OCH_3$ | $-NHCH_3$ | N | O | |
| 2.279 | $-CH_2-CH_2-CH_2-CN$ | 2 | H | $OCH_3$ | $-OCH_2-CF_3$ | N | O | |
| 2.280 | $-CH_2-CH_2-CH_2-CN$ | 2 | H | $OCH_3$ | $C_2H_5$ | N | O | |
| 2.281 | $-CH_2-CH_2-CH_2-CN$ | 2 | H | $OC_2H_5$ | $C_2H_5$ | N | O | |
| 2.282 | $-CH_2-CH_2-CH_2-O-CO-CH_3$ | 2 | H | $CH_3$ | $OCH_3$ | N | O | 134-135 |
| 2.283 | $-CH_2-CH_2-CH_2-O-CO-CH_3$ | 2 | H | $CH_3$ | $OC_2H_5$ | N | O | |
| 2.284 | $-CH_2-CH_2-CH_2-O-CO-CH_3$ | 2 | H | $OCH_3$ | $OCH_3$ | N | O | |
| 2.285 | $-CH_2-CH_2-CH_2-O-CO-CH_3$ | 2 | H | $OCH_3$ | $OC_2H_5$ | N | O | |
| 2.286 | $-CH_2-CH_2-CH_2-O-CO-CH_3$ | 2 | H | $OCH_3$ | $-N(CH_3)_2$ | N | O | |
| 2.287 | $-CH_2-CH_2-CH_2-O-CO-CH_3$ | 2 | H | $OCH_3$ | $-OCH_2-CF_3$ | N | O | |
| 2.288 | $-CH_2-CH_2-CH_2-O-CO-CH_3$ | 2 | H | $OCH_3$ | $-NHCH_3$ | N | O | |
| 2.289 | $-CH_2-CH_2-CH_2-O-CO-CH_3$ | 2 | H | $OCH_3$ | $C_2H_5$ | N | O | |
| 2.290 | $-CH_2-CH_2-CH_2-O-CO-CH_3$ | 2 | H | $OC_2H_5$ | $C_2H_5$ | N | O | |
| 2.291 | $-CH_2-O-CH_2-CH_2-OCH_3$ | 2 | H | $CH_3$ | $CH_3$ | N | O | |
| 2.292 | $-CH_2-O-CH_2-CH_2-OCH_3$ | 2 | H | $CH_3$ | $OCH_3$ | N | O | |
| 2.293 | $-CH_2-O-CH_2-CH_2-OCH_3$ | 2 | H | $OCH_3$ | $OCH_3$ | N | O | |
| 2.294 | $-CH_2-O-CH_2-CH_2-OCH_3$ | 2 | H | $CH_3$ | $OC_2H_5$ | N | O | |
| 2.295 | $-CH_2-O-CH_2-CH_2-OCH_3$ | 2 | H | $OCH_3$ | $OC_2H_5$ | N | O | |
| 2.296 | $-CH_2-O-CH_2-CH_2-OCH_3$ | 2 | H | $OCH_3$ | $C_2H_5$ | N | O | |
| 2.297 | $-CH_2-O-CH_2-CH_2-OCH_3$ | 2 | H | $OC_2H_5$ | $C_2H_5$ | N | O | |
| 2.298 | $-CH_2-O-CH_2-CH_2-OCH_3$ | 2 | H | $OCH_3$ | $-N(CH_3)_2$ | N | O | |
| 2.299 | $-CH_2-O-CH_2-CH_2-OCH_3$ | 2 | H | $OCH_3$ | $-NHCH_3$ | N | O | |
| 2.300 | $-CH_2-O-CH_2-CH_2-OCH_3$ | 2 | H | $OCH_3$ | $-OCH_2-CF_3$ | N | O | |
| 2.301 | $-CH_2-O-CH_2-CH_2-OCH_3$ | 2 | H | $OCH_3$ | $CH_2F$ | N | O | |
| 2.302 | $-CH_2-CH_2-COOC_2H_5$ | 2 | H | $CH_3$ | $OCH_3$ | CH | O | 136-137 |
| 2.303 | $-CH_2-CH_2-COOC_2H_5$ | 2 | H | $CH_3$ | $OCHF_2$ | CH | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.304 | $-CH_2-CH_2-COOC_2H_5$ | 2 | H | $OCH_3$ | $OCH_3$ | CH | O | |
| 2.305 | $-CH_2-CH_2-COOC_2H_5$ | 2 | H | $OCH_3$ | $-N(CH_3)_2$ | CH | O | |
| 2.306 | $-CH_2-CH_2-COOC_2H_5$ | 2 | H | $CH_3$ | $CH_3$ | CH | O | |
| 2.307 | $-CH_2-CH_2-COOC_2H_5$ | 2 | H | $OCH_3$ | Cl | CH | O | |
| 2.308 | $-CH_2-CH_2-COOC_2H_5$ | 2 | H | $OCH_3$ | $OCHF_2$ | CH | O | |
| 2.309 | $-CH_2-CH_2-COOC_3H_7-i$ | 2 | H | $CH_3$ | $OCH_3$ | CH | O | |
| 2.310 | $-CH_2-CH_2-COOC_3H_7-i$ | 2 | H | $CH_3$ | $OCHF_2$ | CH | O | |
| 2.311 | $-CH_2-CH_2-COOC_3H_7-i$ | 2 | H | $CH_3$ | $CH_3$ | CH | O | |
| 2.312 | $-CH_2-CH_2-COOC_3H_7-i$ | 2 | H | $OCH_3$ | $OCH_3$ | CH | O | |
| 2.313 | $-CH_2-CH_2-COOC_3H_7-i$ | 2 | H | $OCH_3$ | Cl | CH | O | |
| 2.314 | $-CH_2-CH_2-COOC_3H_7-i$ | 2 | H | $OCH_3$ | $OCHF_2$ | CH | O | |
| 2.315 | $-CH_2-CH_2-COOC_3H_7-i$ | 2 | H | $OCH_3$ | $-N(CH_3)_2$ | CH | O | |
| 2.316 | $-CH(OC_2H_5)-CH_2-SO_2-CH_3$ | 2 | H | $CH_3$ | $OCH_3$ | N | O | 194-195 |
| 2.317 | $-CH_2-S-CO-C_6H_5$ | 2 | H | $CH_3$ | $OCH_3$ | N | O | |
| 2.318 | $-CH_2-S-CO-C_6H_5$ | 2 | H | $OCH_3$ | $OCH_3$ | N | O | 164-167 |
| 2.319 | $-CH_2-S-CO-C_6H_5$ | 2 | H | $OCH_3$ | $OCH_2-CF_3$ | N | O | |
| 2.320 | $-CH_2-S-CO-C_6H_5$ | 2 | H | $CH_3$ | $CH_3$ | CH | O | |
| 2.321 | $-CH_2-S-CO-C_6H_5$ | 2 | H | $CH_2$ | $OCH_3$ | CH | O | 174-177 |
| 2.322 | $-CH_2-S-CO-C_6H_5$ | 2 | H | $OCH_3$ | $OCH_3$ | CH | O | |
| 2.323 | $-CH_2-S-CO-C_6H_5$ | 2 | H | $OCH_3$ | $OCHF_2$ | CH | O | |
| 2.324 | $-CH_2-S-CO-C_6H_5$ | 2 | H | $OCHF_2$ | $OCHF_2$ | CH | O | |
| 2.325 | $-CH_2-S-CO-C_6H_5$ | 2 | H | $CH_3$ | $OCHF_2$ | CH | O | |
| 2.326 | $-CH_2-S-CO-C_6H_5$ | 2 | H | $OCH_3$ | Cl | CH | O | |
| 2.327 | $-CH_2-S-CO-CH_3$ | 2 | H | $CH_3$ | $OCH_3$ | N | O | 163-166 |
| 2.328 | $-CH_2-S-CO-CH_3$ | 2 | H | $OCH_3$ | $OCH_3$ | N | O | |
| 2.329 | $-CH_2-S-CO-CH_3$ | 2 | H | $OCH_3$ | $-OCH_2CF_3$ | N | O | |
| 2.330 | $-CH_2-S-CO-N(CH_3)_2$ | 2 | H | $CH_3$ | $OCH_3$ | CH | O | 194-197 |
| 2.331 | $-CH_2-S-CO-N(CH_3)_2$ | 2 | H | $OCH_3$ | $OCH_3$ | CH | O | |

Tabelle 2 (Fortsetzung)

| Nr. | A | Stellung von A | $R^1$ | $R^3$ | $R^4$ | E | Z | Smp. [°C] |
|---|---|---|---|---|---|---|---|---|
| 2.332 | $-CH_2-S-CO-N(CH_3)_2$ | 2 | H | $CH_3$ | $CH_3$ | CH | O | |
| 2.333 | $-CH_2-S-CO-N(CH_3)_2$ | 2 | H | $OCHF_2$ | $OCHF_2$ | CH | O | |
| 2.334 | $-CH_2-S-CO-N(CH_3)_2$ | 2 | H | $CH_3$ | $OCH_3$ | N | O | 193-195 |
| 2.335 | $-CH_2-S-CO-N(CH_3)_2$ | 2 | H | $OCH_3$ | $OCH_3$ | N | O | |
| 2.336 | $-CH_2-S-CO-N(CH_3)_2$ | 2 | H | $OCH_3$ | $-OCH_2-CF_3$ | N | O | |
| 2.337 | $-CH_2-S-CO-N(CH_3)OCH_3$ | 2 | H | $CH_3$ | $OCH_3$ | N | O | |
| 2.338 | $-CH_2-S-CO-N(CH_3)OCH_3$ | 2 | H | $OCH_3$ | $OCH_3$ | N | O | |
| 2.339 | $-CH_2-S-CO-N(CH_3)OCH_3$ | 2 | H | $CH_3$ | $OCH_3$ | CH | O | 177-178 (Zers.) |
| 2.340 | $-CH_2-S-CO-N(CH_3)OCH_3$ | 2 | H | $OCH_3$ | $OCH_3$ | CH | O | |
| 2.341 | $-CH_2-S-CO-N(CH_3)OCH_3$ | 2 | H | $CH_3$ | $CH_3$ | CH | O | |
| 2.342 | $-CH_2-S-CO-N(CH_3)OCH_3$ | 2 | H | $OCHF_2$ | $OCHF_2$ | CH | O | |

Formulierungsbeispiele

Beispiel F1: Formulierungsbeispiele für Wirkstoffe der Formel I

(% = Gewichtsprozent

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykoläther (7-8 Mo AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1% | 1% |
| Talkum | 99,9% | - |
| Kaolin | - | 99% |

Man erhält anwendungsfertige Stäubemittel, indem der Wirkstoff mit dem Träger vermischt und auf einer geeigneten Mühle vermahlen wird.

d) Extruder-Granulat

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungsgranulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |

| | a) | b) |
|---|---|---|
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel B1: Herbizidwirkung vor dem Auflaufen der Pflanzen

Kunststofftöpfe werden mit expandiertem Vermiculit (Dichte: 0,135 $g/cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr. | Nasturtium | Stellaria | Agrostis | Digitaria |
|---|---|---|---|---|
| 2.1 | 2 | 2 | 1 | 2 |
| 2.22 | 2 | 1 | 2 | 2 |
| 2.73 | 2 | 1 | 1 | 2 |
| 2.87 | 2 | 2 | 2 | 2 |
| 2.129 | 2 | 3 | 2 | 3 |
| 2.165 | 2 | 2 | 2 | 2 |
| 2.183 | 2 | 1 | 1 | 2 |
| 2.237 | 5 | 4 | 8 | 7 |
| 2.282 | 2 | 2 | 2 | 4 |
| 2.318 | 1 | 1 | 1 | 2 |
| 2.321 | 1 | 1 | 1 | 2 |
| 2.327 | 2 | 2 | 2 | 3 |
| 2.330 | 1 | 1 | 1 | 2 |
| 2.334 | 2 | 2 | 2 | 2 |
| 2.339 | 2 | 1 | 2 | 2 |

Beispiel B2: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen), ohne dass dabei die Versuchspflanzen geschädigt wurden.

Beispiel B3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B4: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca.

21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90% der Kontrolle), sowie teilweise eine Zunahme der Stengeldurchmesser auf.

Beispiel B5: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchtentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30% im Vergleich zur unbehandelten Kontrolle.

Patentansprüche für die Vertragsstaaten: BE, CH, LI, DE, FR, GB, IT, NL

1. N-Phenylsulfonyl-N'-pyrimidinyl- und -triazinylharnstoffe der Formel I

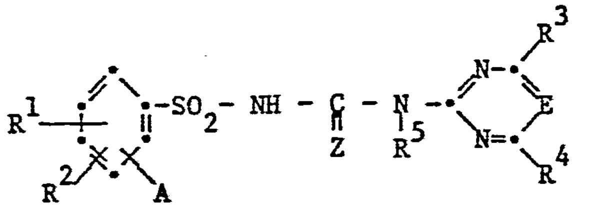

(I),

worin

A einen Rest der Formeln $-CR^6R^7-XR^8$, $-CR^9R^{10}R^{11}$ oder $-CHR^7-S-CQ-R^{21}$,

$R^1$ Wasserstoff, Halogen, Nitro, Cyan, $C_1$-$C_4$-Alkyl, $C_1$-$C_4$-Halogenalkyl, $-Y-R^{14}$, $-CO-NR^{12}R^{13}$, $-NR^{12}R^{13}$, $-SO-NR^{15}R^{16}$, $-O-SO_2-R^{17}$ oder $-CO-R^{18}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_5$-Alkoxyalkyl oder $-NR^{19}R^{20}$,

$R^5$ Wasserstoff, $C_1$-$C_4$-Alkyl oder $C_1$-$C_4$-Alkoxy,

$R^6$ Wasserstoff, $C_1$-$C_4$-Alkyl oder Fluor,

$R^7$ Wasserstoff oder Methyl,

$R^8$ $C_1$-$C_4$-Halogenalkyl mit höchstens 2 Halogenatomen, $C_2$-$C_5$-Alkoxyalkyl, $C_2$-$C_5$-Halogenalkenyl, $C_3$-$C_5$-Alkinyl oder $C_1$-$C_4$-Cyanoalkyl,

$R^9$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^{10}$ Wasserstoff, Halogen oder Methyl,

$R^{11}$ einen Rest $-CO-R^{24}$ oder einen einfach oder mehrfach durch Substituenten aus der Gruppe Cyan, Nitro, Hydroxyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $-CO-R^{18}$, $-S-CO-R^{18}$, $-O-CO-R^{18}$, $-NR^{12}R^{13}$, $-CO-NR^{12}R^{13}$, $-O-SO_2-R^{17}$ oder $-SO_2-NR^{15}R^{16}$ substitu-

ierten $C_1$-$C_4$-Alkylrest, der zusätzlich durch ein oder mehrere Halogenatome substituiert sein kann,

$R^{12}$ und $R^{13}$ unabhängig voneinander Wasserstoff oder $C_1$-$C_4$-Alkyl,

$R^{14}$ $C_2$-$C_5$-Alkenyl, $C_2$-$C_5$-Halogenalkenyl, $C_1$-$C_4$-Alkyl, $C_1$-$C_4$-Halogenalkyl oder $C_2$-$C_5$-Alkoxyalkyl,

$R^{15}$ Wasserstoff, $C_1$-$C_4$-Alkyl oder Methoxy,

$R^{16}$ Wasserstoff, Methyl oder Aethyl,

$R^{17}$ $C_1$-$C_4$-Alkyl, $C_1$-$C_4$-Halogenalkyl oder $-NR^{12}R^{13}$,

$R^{18}$ Wasserstoff, $C_1$-$C_4$-Alkoxy, $C_1$-$C_4$-Halogenalkoxy, $C_1$-$C_4$-Alkylthio, $C_1$-$C_4$-Alkyl, $C_1$-$C_4$-Halogenalkyl oder $C_2$-$C_5$-Alkoxyalkoxy,

$R^{19}$ und $R^{20}$ unabhängig voneinander Wasserstoff oder $C_1$-$C_4$-Alkyl,

$R^{21}$ $C_1$-$C_4$-Alkyl, $C_1$-$C_4$-Alkoxy, $C_1$-$C_4$-Alkylthio, $-NR^{22}R^{23}$ oder gegebenenfalls durch Halogen oder $C_1$-$C_4$-Alkyl substituiertes Phenyl,

$R^{22}$ $C_1$-$C_4$-Alkyl oder Methoxy,

$R^{23}$ $C_1$-$C_4$-Alkyl,

$R^{22}$ und $R^{23}$ zusammen eine 4 bis 5 gliedrige, gegebenenfalls durch Sauerstoff oder $-NCH_3-$ unterbrochene Alkylenbrücke,

$R^{24}$ Wasserstoff, $C_1$-$C_4$-Alkyl oder $C_1$-$C_4$-Halogenalkyl,

E Stickstoff oder die Methylenbrücke,

Q und Z unabhängig voneinander Sauerstoff oder Schwefel und

Y und X unabhängig voneinander Sauerstoff, Schwefel, die Sulfinyl- oder Sulfonylbrücke bedeuten, sowie die Salze dieser Verbindungen.

2. Verbindungen gemäss Anspruch 1, dadurch gekennzeichnet, dass $R^1$ Wasserstoff bedeutet.

3. Verbindungen gemäss Anspruch 1, dadurch gekennzeichnet, dass $R^2$ Wasserstoff bedeutet.

4. Verbindungen gemäss Anspruch 1, dadurch gekennzeichnet, dass $R^5$ Wasserstoff bedeutet.

5. Verbindungen gemäss Anspruch 1, dadurch gekennzeichnet, dass $R^3$ und $R^4$ zusammen höchstens 4 Kohlenstoffatome enthalten.

6. Verbindungen gemäss Anspruch 1, dadurch gekennzeichnet, dadurch gekennzeichnet, dass Z Sauerstoff bedeutet.

7. Verbindungen gemäss Anspruch 1, dadurch gekennzeichnet, dass der Rest A die 2-Stellung des Phenylkerns besetzt.

8. Verbindungen gemäss Anspruch 1, dadurch gekennzeichnet, dass die Reste $R^6$, $R^7$, $R^9$ und $R^{10}$ Wasserstoff bedeuten.

9. Verbindungen gemäss Anspruch 1, dadurch gekennzeichnet, dass $R^1$, $R^2$ und $R^5$ Wasserstoff bedeuten, $R^3$ und $R^4$ zusammen höchstens 4 Kohlenstoffatome enthalten und Z Sauerstoff ist.

10. Verbindungen gemäss Anspruch 7, dadurch gekennzeichnet, dass der Rest A ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

11. Verbindungen gemäss Anspruch 1, dadurch gekennzeichnet, dass die Reste $R^6$, $R^7$, $R^9$ und $R^{10}$ Wasserstoff bedeuten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

12. Verbindungen gemäss Anspruch 1, dadurch gekennzeichnet, dass die Reste $R^1$, $R^2$, $R^5$, $R^6$, $R^7$, $R^9$ und $R^{10}$ Wasserstoff und Z Sauerstoff bedeuten, die Reste $R^3$ und $R^4$ zusammen höchstens 4 Kohlenstoffatome enthalten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

13. N-[2-(3-Oxobutyl)-phenylsulfonyl]-N'-(4-methoxy-6-methyl-1,3,5-triazin-2-yl)-harnstoff gemäss Anspruch 1.

14. Phenylsulfonamide der Formel II

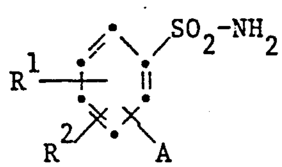

(II)

worin $R^1$, $R^2$ und A die unter Formel I im Anspruch 1 gegebene Bedeutung haben.

15. Phenylsulfonylisocyanate und -isothiocyanate der Formel IV

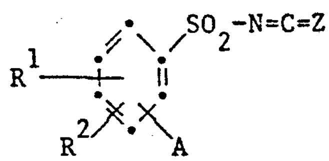

(IV)

worin $R^1$, $R^2$, A und Z die unter Formel I in Anspruch 1 gegebene Bedeutung haben.

16. N-Phenylsulfonylcarbamate der Formel VII

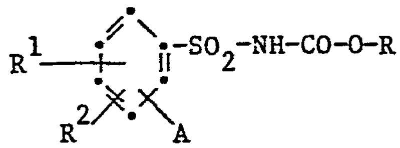

(VII)

worin $R^1$, $R^2$ und A die unter Formel I in Anspruch 1 gegebene Bedeutung haben und R für Phenyl, Alkyl oder substituiertes Phenyl steht.

17. Verfahren zur Herstellung der Verbindungen der Formel I, dadurch gekennzeichnet, dass man ein Phenylsulfonamid der Formel II

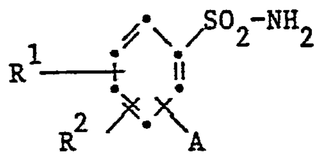

(II),

worin A, $R^1$ und $R^2$ die unter Formel I gegebene Bedeutung haben, in Gegenwart einer Base mit einem N-Pyrimidinyl- oder -Triazinylcarbamat der Formel III

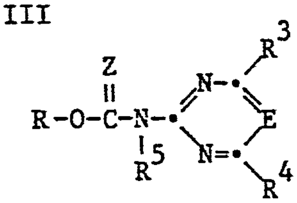

(III),

worin E, $R^3$, $R^4$, $R^5$ und Z die unter Formel I gegebene Bedeutung haben und R für Phenyl, Alkyl oder substituiertes Phenyl steht, umsetzt und gegebenenfalls in ihre Salze überführt.

18. Verfahren zur Herstellung der Verbindungen der Formel I, dadurch gekennzeichnet, dass man ein Phenylsulfonylisocyanat oder -isothiocyanat der Formel IV

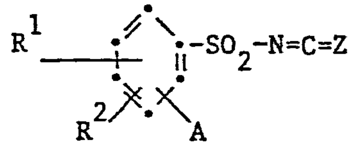

(IV),

worin

A, $R^1$, $R^2$ und Z die unter Formel I gegebene Bedeutung haben, gegebenenfalls in Gegenwart einer Base, mit einem Amin der Formel V

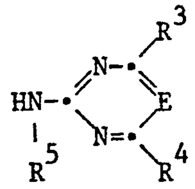

(V),

worin E, $R^3$, $R^4$ und $R^5$ die unter Formel I gegebene Bedeutung haben, umsetzt und gegebenenfalls in ihre Salze überführt.

19. Verfahren zur Herstellung der Verbindungen der Formel I in denen $R^5$ Wasserstoff ist, dadurch gekennzeichnet, dass man ein Sulfonamid der Formel II gemäss Anspruch 17 gegebenenfalls in Gegenwart einer Base mit einem Isocyanat oder Isothiocyanat der Formel VI

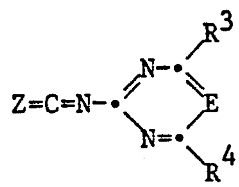

(VI),

worin
E, $R^3$, $R^4$, $R^5$ und Z die unter Formel I gegebene Bedeutung haben, umsetzt und gegebenenfalls in ihre Salze überführt.

20. Verfahren zur Herstellung der Verbindungen der Formel I, dadurch gekennzeichnet, dass man ein N-Phenylsulfonylcarbamat der Formel VII

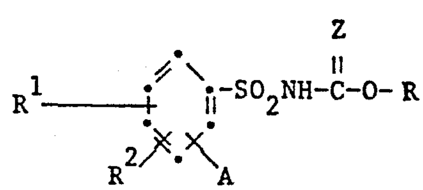

(VII) ,

worin A, $R^1$, $R^2$ und Z die unter Formel I gegebene Bedeutung haben und R für Phenyl, Alkyl oder substituiertes Phenyl steht, mit einem Amin der Formel V gemäss Anspruch 18 umsetzt und gegebenenfalls in ihre Salze überführt.

21. Verfahren zur Herstellung von Additionssalzen der Formel I gemäss einem der Ansprüche 17 bis 20, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

22. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I, Anspruch 1, enthält.

23. Die Verwendung der N-Phenylsulfonyl-N'-triazinyl- oder -pyrimidinyl-harnstoffe der Formel I, Anspruch 1, oder sie enthaltender Mittel zur Bekämpung unerwünschten Pflanzenwachstums.

24. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

25. Die Verwendung der N-Phenylsulfonyl-N'-triazinyl- oder -pyrimidinyl-harnstoffe der Formel I, oder sie enthaltender Mittel, gemäss Anspruch 23, zur selektiven pre-oder postemergenten Bekämpfung von Unkräutern in Nutzpflanzkulturen.

26. Die Verwendung der N-Phenylsulfonyl-N'-triazinyl- oder -pyrimidinyl-harnstoffe der Formel I, oder sie enthaltender Mittel, gemäss Anspruch 24, zur Unterdrückung des Pflanzenwachstums über das 2-Blattstadium hinaus, dadurch gekennzeichnet, dass die Wirkstoffe preemergent angewendet werden.

27. Die Verwendung gemäss Anspruch 25 in Zuckerrohr-, Getreide-, Mais-, Soja-, Reis- und Baumwollkulturen.

28. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

29. Die Verwendung gemäss Anspruch 28 in Sojakulturen.

30. Die Verwendung gemäss Anspruch 24 bei Bodendecker-Leguminosen.

Patentansprüche für den Vertragsstaat AT

1. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I

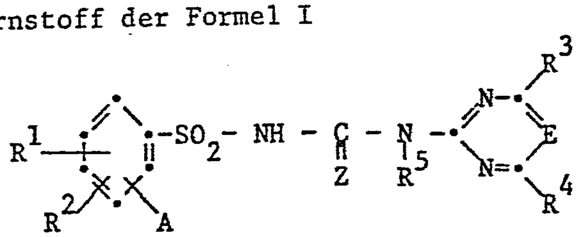

(I),

oder ein Salz davon enthält, worin

A einen Rest der Formeln $-CR^6R^7-XR^8$, $-CR^9R^{10}R^{11}$ oder $-CHR^7-S-CQ-R^{21}$,

$R^1$ Wasserstoff, Halogen, Nitro, Cyan, $C_1$-$C_4$-Alkyl, $C_1$-$C_4$-Halogenalkyl, $-Y-R^{14}$, $-CO-NR^{12}R^{13}$, $-NR^{12}R^{13}$, $-SO-NR^{15}R^{16}$, $-O-SO_2-R^{17}$ oder $-CO-R^{18}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_5$-Alkoxyalkyl oder $-NR^{19}R^{20}$,

$R^5$ Wasserstoff, $C_1$-$C_4$-Alkyl oder $C_1$-$C_4$-Alkoxy,

$R^6$ Wasserstoff, $C_1$-$C_4$-Alkyl oder Fluor,

$R^7$ Wasserstoff oder Methyl,

$R^8$ $C_1$-$C_4$-Halogenalkyl mit höchstens 2 Halogenatomen, $C_2$-$C_5$-Alkoxyalkyl, $C_2$-$C_5$-Halogenalkenyl, $C_3$-$C_5$-Alkinyl oder $C_1$-$C_4$-Cyanoalkyl,

$R^9$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^{10}$ Wasserstoff, Halogen oder Methyl,

$R^{11}$ einen Rest $-CO-R^{24}$ oder einen einfach oder mehrfach durch Substituenten aus der Gruppe Cyan, Nitro, Hydroxyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $-CO-R^{18}$, $-S-CO-R^{18}$, $-O-CO-R^{18}$, $-NR^{12}R^{13}$, $-CO-NR^{12}R^{13}$, $-O-SO_2-R^{17}$ oder $-SO_2-NR^{15}R^{16}$ substitu-

ierten $C_1$-$C_4$-Alkylrest, der zusätzlich durch ein oder mehrere Halogenatome substituiert sein kann,

$R^{12}$ und $R^{13}$ unabhängig voneinander Wasserstoff oder $C_1$-$C_4$-Alkyl,

$R^{14}$ $C_2$-$C_5$-Alkenyl, $C_2$-$C_5$-Halogenalkenyl, $C_1$-$C_4$-Alkyl, $C_1$-$C_4$-Halogenalkyl oder $C_2$-$C_5$-Alkoxyalkyl,

$R^{15}$ Wasserstoff, $C_1$-$C_4$-Alkyl oder Methoxy,

$R^{16}$ Wasserstoff, Methyl oder Aethyl,

$R^{17}$ $C_1$-$C_4$-Alkyl, $C_1$-$C_4$-Halogenalkyl oder $-NR^{12}R^{13}$,

$R^{18}$ Wasserstoff, $C_1$-$C_4$-Alkoxy, $C_1$-$C_4$-Halogenalkoxy, $C_1$-$C_4$-Alkylthio, $C_1$-$C_4$-Alkyl, $C_1$-$C_4$-Halogenalkyl oder $C_2$-$C_5$-Alkoxyalkoxy,

$R^{19}$ und $R^{20}$ unabhängig voneinander Wasserstoff oder $C_1$-$C_4$-Alkyl,

$R^{21}$ $C_1$-$C_4$-Alkyl, $C_1$-$C_4$-Alkoxy, $C_1$-$C_4$-Alkylthio, $-NR^{22}R^{23}$ oder gegebenenfalls durch Halogen oder $C_1$-$C_4$-Alkyl substituiertes Phenyl,

$R^{22}$ $C_1$-$C_4$-Alkyl oder Methoxy,

$R^{23}$ $C_1$-$C_4$-Alkyl,

$R^{22}$ und $R^{23}$ zusammen eine 4 bis 5 gliedrige, gegebenenfalls durch Sauerstoff oder $-NCH_3-$ unterbrochene Alkylenbrücke,

$R^{24}$ Wasserstoff, $C_1$-$C_4$-Alkyl oder $C_1$-$C_4$-Halogenalkyl,

E Stickstoff oder die Methylenbrücke,

Q und Z unabhängig voneinander Sauerstoff oder Schwefel und

Y und X unabhängig voneinander Sauerstoff, Schwefel, die Sulfinyl- oder Sulfonylbrücke bedeuten.

2. Mittel gemäss Anspruch 1, dadurch gekennzeichnet, dass $R^1$ Wasserstoff bedeutet.

3. Mittel gemäss Anspruch 1, dadurch gekennzeichnet, dass $R^2$ Wasserstoff bedeutet.

4. Mittel gemäss Anspruch 1, dadurch gekennzeichnet, dass $R^5$ Wasserstoff bedeutet.

5. Mittel gemäss Anspruch 1, dadurch gekennzeichnet, dass $R^3$ und $R^4$ zusammen höchstens 4 Kohlenstoffatome enthalten.

6. Mittel gemäss Anspruch 1, dadurch gekennzeichnet, dadurch gekennzeichnet, dass Z Sauerstoff bedeutet.

7. Mittel gemäss Anspruch 1, dadurch gekennzeichnet, dass der Rest A die 2-Stellung des Phenylkerns besetzt.

8. Mittel gemäss Anspruch 1, dadurch gekennzeichnet, dass die Reste $R^6$, $R^7$, $R^9$ und $R^{10}$ Wasserstoff bedeuten.

9. Mittel gemäss Anspruch 1, dadurch gekennzeichnet, dass $R^1$, $R^2$ und $R^5$ Wasserstoff bedeuten, $R^3$ und $R^4$ zusammen höchstens 4 Kohlenstoffatome enthalten und Z Sauerstoff ist.

10. Mittel gemäss Anspruch 7, dadurch gekennzeichnet, dass der Rest A ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

11. Mittel gemäss Anspruch 1, dadurch gekennzeichnet, dass die Reste $R^6$, $R^7$, $R^9$ und $R^{10}$ Wasserstoff bedeuten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

12. Verbindungen gemäss Anspruch 1, dadurch gekennzeichnet, dass die Reste $R^1$, $R^2$, $R^5$, $R^6$, $R^7$, $R^9$ und $R^{10}$ Wasserstoff und Z Sauerstoff bedeuten, die Reste $R^3$ und $R^4$ zusammen höchstens 4 Kohlenstoffatome enthalten und der Rest A die 2-Stellung des Phenylkerns besetzt und ausgewählt ist aus der Gruppe 3-Cyanopropyl, 2-Cyanoäthyl, 3-Acetoxypropyl, 3-Acetoxybutyl, 2-Methoxycarbonyläthyl, 2-Aethoxycarbonylpropyl, 2-Aethoxycarbonyläthyl, 2-Methoxyäthyl, 3-Methoxypropyl, 2-Hydroxyläthyl, 3-Hydroxylpropyl, 3-Hydroxylbutyl, 2-Methylcarbonyläthyl, Difluormethoxymethyl, (2-Methoxyäthoxy)-methyl, (2-Methoxyäthylthio)-methyl oder Methylthiocarbonylthiomethyl.

13. Mittel gemäss Anspruch 1, dadurch gekennzeichnet, dass es als Wirkstoff N-[2-(3-Oxobutyl)-phenylsulfonyl]-N'-(4-methoxy-6-methyl-1,3,5-triazin-2-yl)-harnstoff enthält.

14. Verfahren zur Herstellung der Verbindungen der Formel I, dadurch gekennzeichnet, dass man

a) entweder ein Phenylsulfonamid der Formel II

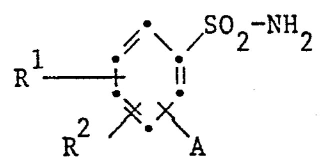

(II),

worin A, $R^1$ und $R^2$ die unter Formel I gegebene Bedeutung haben, in Gegenwart einer Base mit einem N-Pyrimidinyl- oder -Triazinylcarbamat der Formel III

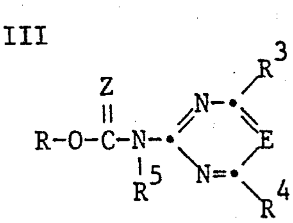

(III),

worin E, $R^3$, $R^4$, $R^5$ und Z die unter Formel I gegebene Bedeutung haben und R für Phenyl, Alkyl oder substituiertes Phenyl steht, umsetzt oder

b) ein Phenylsulfonylisocyanat oder -isothiocyanat der Formel IV

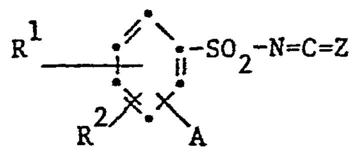

(IV),

worin
A, $R^1$, $R^2$ und Z die unter Formel I gegebene Bedeutung haben, gegebeenenfalls in Gegenwart einer Base, mit einem Amin der Formel V

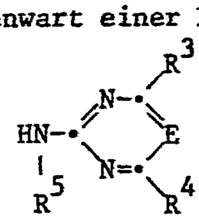

(V),

worin E, $R^3$, $R^4$ und $R^5$ die unter Formel I gegebene Bedeutung haben, umsetzt oder

c) ein N-Phenylsulfonylcarbamat der Formel VII

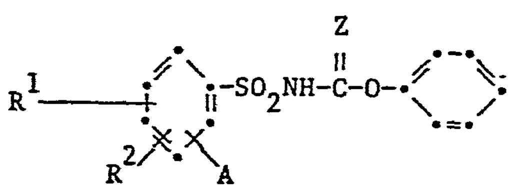

(VII) ,

worin A, $R^1$, $R^2$ und Z die unter Formel I gegebene Bedeutung haben, mit einem Amin der Formel V umsetzt und gegebenenfalls in ihre Salze überführt indem man einen Sulfonylharnstoff der Formel I mit einem Amin, einem Alkalimetall- oder Erdalkalimetallhydroxid oder einer quaternären Ammoniumbase umsetzt.

15. Die Verwendung der N-Phenylsulfonyl-N'-triazinyl- oder -pyrimidinyl-harnstoffe der Formel I, nach Anspruch 1, oder sie enthaltender Mittel zur Bekämpfung unerwünschten Pflanzenwachstums.

16. Die Verwendung der N-Phenylsulfonyl-N'-triazinyl- oder -pyrimidinyl-harnstoffe der Formel I, nach Anspruch 1, oder sie enthaltender Mittel zur Regulierung des Pflanzenwachstums.

17. Die Verwendung gemäss Anspruch 15, zur selektiven pre- oder postemergenten Bekämpfung von Unkräutern in Nutzpflanzenkulturen.

18. Die Verwendung gemäss Anspruch 16, zur Unterdrückung des Pflanzenwachstums über das 2-Blattstadium hinaus, dadurch gekennzeichnet, dass die Wirkstoffe preemergent angewendet werden.

19. Die Verwendung gemäss Anspruch 17 in Zuckerrohr-, Getreide-, Mais-, Soja-, Reis- und Baumwollkulturen.

20. Die Verwendung der N-Phenylsulfonyl-N'-triazinyl- oder -pyrimidinyl-harnstoffe der Formel I, nach Anspruch 1, oder sie enthaltender Mittel zur Wuchsregulierung von Kulturpflanzen zum Zwecke einer Ertragssteigerung.

21. Die Verwendung gemäss Anspruch 20 in Sojakulturen.

22. Die Verwendung gemäss Anspruch 16 bei Bodendecker-Leguminosen.

FO 7.5 CW/gs*/rz*/cw*/rn*

Europäisches Patentamt

**EUROPÄISCHER RECHERCHENBERICHT**

**0125205**
Nummer der Anmeldung

EP 84 81 0180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | | | C 07 D 239/46<br>C 07 D 251/16<br>C 07 D 251/46<br>C 07 C 143/78<br>C 07 C 143/828<br>C 07 C 143/83<br>A 01 N 47/36 |
| Y | EP-A-0 044 209 (E.I. DU PONT DE NEMOURS AND COMPANY)<br>* Ansprüche 1,17,18; Beispiele 16,32,33; Seiten 85-91 *<br>--- | 1,14, 15,22 | |
| Y | EP-A-0 046 677 (E.I. DU PONT DE NEMOURS AND COMPANY)<br>* Ansprüche *<br>--- | 1,22 | |
| D,A | EP-A-0 044 807 (CIBA-GEIGY AG)<br>* Ansprüche *<br>----- | 1,22 | |
| | | | **RECHERCHIERTE SACHGEBIETE (Int. Cl. 3)** |
| | | | C 07 D 239/00<br>C 07 D 251/00<br>C 07 C 143/00 |

Der vorliegende Recherchenbericht wurde für alle Patentansprüche erstellt.

| Recherchenort | Abschlußdatum der Recherche | Prüfer |
|---|---|---|
| DEN HAAG | 13-07-1984 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82